# EUROPÄISCHE PATENTSCHRIFT

(11) **EP 0 929 677 B1**
(45) Veröffentlichungstag und Bekanntmachung des Hinweises auf die Patenterteilung: **20.07.2005**
(21) Anmeldenummer: 97911173.9
(22) Anmeldetag: 02.10.1997
(51) Int. Cl.: C12N 15/30, C07K 14/445, C12N 15/62, A61K 39/015, A61K 31/70, C07H 21/00, C12N 1/21, C12N 5/10, C12N 1/11, C12N 15/67

(54) **REKOMBINANTES HERSTELLUNGSVERFAHREN FÜR EIN VOLLSTÄNDIGES MALARIA-ANTIGEN GP190/MSP1**
RECOMBINANT METHOD FOR PREPARING A COMPLETE MALARIA ANTIGEN GP190/MSP1
METHODE RECOMBINANTE POUR LA PREPARATION D'UN ANTIGENE COMPLET DE MALARIA GP190/MSP1

(30) Priorität: 02.10.1996 DE 19640817
(43) Veröffentlichungstag der Anmeldung: 21.07.1999
(73) Patentinhaber: Bujard, Hermann, 69120 Heidelberg (DE)
(72) Erfinder: BUJARD, Hermann, D-69120 Heidelberg (DE); TOLLE, Ralf, D-71636 Ludwigsburg (DE); PAN, Weiqing, D-69123 Heidelberg (DE)
(74) Vertreter: Grünecker, Kinkeldey, Stockmair & Schwanhäusser Anwaltssozietät
(86) Internationale Anmeldenummer: PCT/EP1997/005441
(87) Internationale Veröffentlichungsnummer: WO 1998/014583

(56) Entgegenhaltungen:
- EP-A- 0 154 454
- EP-A- 0 340 359
- EP-A- 0 359 472
- EP-A- 0 385 962
- WO-A-94/28930
- KASLOW DC ET AL: "Expression and antigenicity of Plasmodium falciparum major merozoite surface protein (MSP1(19)) variants secreted from Saccharomyces cerevisiae." MOL BIOCHEM PARASITOL, FEB 1994, 63 (2) P283-9, NETHERLANDS, XP000603953
- HOLDER A A ET AL: "PRIMARY STRUCTURE OF THE PRECURSOR TO THE THREE MAJOR SURFACE ANTIGENS OF PLASMODIUM FALCIPARUM MEROZOITES" NATURE, Bd. 317, 19.September 1985, Seiten 270-273, XP000604859
- MYLER P J: "Nucleotide and deduced amino acid sequence of the gp195 (MSA-1) gene from Plasmodium falciparum Palo Alto PLF-3/B11" NUCLEIC ACIDS RESEARCH., Bd. 17, Nr. 13, 1989, OXFORD GB, Seite 5401 XP002057620
- SIDDIQUI W A ET AL: "Merozoite surface coat precursor protein completely protects Aotus monkeys against Plasmodium falciparum malaria" PROCEEDINGS OF THE NATIONAL ACADEMY OF SCIENCES OF USA., Bd. 84, 1987, WASHINGTON US, Seiten 3014-3018, XP002057621 in der Anmeldung erwähnt
- GENTZ R ET AL: "Major surface antigen p190 of Plasmodium falciparum: detection of common epitopes present in a variety of plasmodia isolates" EMBO JOURNAL., Bd. 7, Nr. 1, 1988, EYNSHAM, OXFORD GB, Seiten 225-230, XP002057622
- PAN W ET AL: "A direct and rapid sequencing strategy for the Plasmodium falciparum antigen gene gp190/MSA1." MOL BIOCHEM PARASITOL, JUL 1995, 73 (1-2) P241-4, NETHERLANDS, XP002057623

## Beschreibung

Die Erfindung betrifft ein rekombinantes Herstellungsverfahren für das vollständige Malaria-Antigen gp190/MSP1 sowie einzelner natürlich vorkommender Domänen und Teile derselben durch Expression (einer) synthetischer DNA-Sequenzen. Die Erfindung betrifft außerdem die durch das Verfahren hergestellten DNA-Sequenzen und die für die Expression der DNA-Sequenzen verwendeten Wirtsorganismen. Femer betrifft die Erfindung die Verwendung des vollständigen Malaria-Antigens sowie Teile derselben als Impfstoff zur Immunisierung gegen Malaria.

Malaria ist weltweit eine der bedeutendsten Infektionskrankheiten. Nach Angaben der WHO waren 1990 in 99 Ländern 40% der Weltbevölkerung einem Malariarisiko ausgesetzt. Ihre Verbreitung nimmt derzeit wieder massiv zu. Dies ist vor allem auf eine intensive Resistenzbildung der Malariaerreger zurückzuführen, die dadurch gefördert wird, daß die zur Therapie eingesetzten Medikamente ebenfalls als Prophylaxe empfohlen und eingenommen werden. Neben der Suche nach neuen, wirksamen Chemotherapeutika werden heute Hoffnungen auch in die Entwicklung von Impfstoffen gesetzt, da Menschen in Malaria-epidemischen Regionen der Welt verschiedene Arten von Immunität zu entwickeln vermögen. Neben einer natürlichen Resistenz gegen Malaria, die sich bei heterozygoten Trägern des Sichelzellgens und bei Personen mit Thalassämie und Gluccse-6-Phosphat-Dehydrogenase-Mangel ausbildet, können im Laufe einer Malariainfektion im Menschen Immunitätsmechanismen einsetzen, die sich in einer erhöhten Widerstandsfähigkeit gegenüber den Plasmodien äußem: Dementsprechend ist der Krankheitsverlauf in stark durchseuchten Bevöikerungspopulationen weitaus weniger bedrohlich als bei Personen, die der Infektion weniger häufig oder erstmalig ausgesetzt sind.

Das Hauptproblem bei der Impfstoffentwicklung ist die Identifikation eines Antigens, das schützende Immunität bewirken kann, da kein leicht zugängliches und gut definiertes Tiermodell für die vier den Menschen befallenden Parasiten vorhanden ist. Die Malaria-Erreger gehören der Gruppe der Plasmodien an, wobei die Infektion mit einem der vier Erreger Plasmodium vivax, Plasmodium ovale, Plasmodium malariae oder *Plasmodium falciparum* durch den Stich von Anopheles-Mücken erfolgt. Von diesem Erreger ist Plasmodium falciparum der gefährlichste und der am weitesten verbreitete.

Das Hauptoberflächenprotein von Merozoiten, der invasiven Form der Blutstadien des Malaria-Erregers Plasmodium falciparum und anderer Malaria-Erreger wie *P*. *vivax*, ist ein 190 - 220 kD Glykoprotein. Spät in der Entwicklung des Parasiten wird dieser Vorläufer in kleinere Proteine prozessiert, die jedoch als einheitlicher Komplex aus Merozoiten isoliert werden können. Der Komplex ist mittels eines Glycosylphosphatidyl-Inositol-Ankers mit der Merozoitenmembran verknüpft. Die Sequenzen der gp 190-Proteine verschiedener *P*. *falciparum*-Stämme fallen in zwei Gruppen, zwischen denen intragene Rekombination häufig ist. Insgesamt besteht das Protein aus mehreren hochkonservierten Regionen, aus einem dimorphem Bereich, welche jeweils einem von zwei Allelen angehören und aus zwei relativ kleinen oligomorphen Blöcken im N-terminalen Bereich (Tanabe, K., Mackay, M., Goman, M. und Scaife, J.G. (1987), Allelic dimorphism in a surface antigen gene of the malaria parasite *Plasmodium falciparum*. J. Mol. Biol. 195, 273-287; Miller, L.H., Roberts, T., Shahabuddin, M. und McCutchan, T.F. (1993), Analysis of sequence diversity in the Plasmodium falciparum merozoite surface protein-1 (MSP-1). Mol. Biochem. Parasitol. 59, 1-14).

Das gp190/MSP1 galt bereits früh als ein möglicher Kandidat für einen Impfstoff. So wurde im Nagermodell nach Immunisierung mit dem analogen Protein aktiver Schutz gegen Infektion mit Nager-Parasiten erhalten. Passiver Schutz ließ sich mit gegen dieses Protein gerichteten Antikörpern erreichen (siehe auch Holder, A. A. und Freeman, R.R. (1981), Immunization against blood-stage rodent malaria using purified parasite antigens, Nature 294, 361-364; Majahan, W.R., Daly, T. M., Weidanz, W.P. und Long, C.A. (1984), Passive immunization against murine malaria with an IgG3 monoclonal antibody, J. Immunol. 132, 3131-3137). Die Daten, die diese Annahme belegen sollen, sind im einzelnen jedoch statistisch nicht signifikant.

Darüber hinaus sind mehrere monoklonale Antikörper, welche in vitro die Invasion von Erythrozyten durch *P*. *falciparum* inhibieren, sind gegen gp190/MSP1 gerichtet (Pirson, P.J. und Perkins, M.E. (1985), Characterization with monoclonal antibodies of a surface antigen of *Plasmodium falciparum* merozoites. J. Immunol. 134, 1946-1951; Blackman, M.J., Heidrich, H.-G., Donachie, S., McBride, J.S. und Holder A.A. (1990), A single fragment of a malaria merozoite surface protein remains on the parasite during red cell invasion and is the target of invasion-inhibiting antibodies, J. Exp. Med. 172, 379-382).

Schließlich wurde eine Reihe von Impfstudien mit gp190/MSP1-Material aus *P. falciparum* an Primaten, insbesondere an Aotus und Saimiri-Affen durchgeführt (siehe auch Perrin, L.H., Merkli, B., Loche, M., Chizzolini, C., Smart, J. und Richle, R. (1984), Antimaiarial immunity in Saimiri monkeys. Immunization with surface components of asexual blood stages, J. Exp. Med. 160,441-451; Hall, R., Hyde, J.E., Goman, M., Simmons, D.L., Hope, I.A., Mackay, M. und Scaife, J.G. (1984), Major surface antigen gene of a human malaria parasite cloned and expressed in bacteria, Nature 311, 379-382; Siddiqui, W.A., Tam, L.O., Kramer, K.J., Hui, G.S.N., Case, S.E., Yamaga, K.M., Chang, S.P., Chan, E.B.T. und Kan, S.-C. (1987), Merozoite surface coat precursor protein completely protects Aotus monkeys against *Plasmodium falciparum* malaria, Proc. Natl. Acad. Sci, USA 84, 3014-3018; Ettlinger, H.M., Caspers, P., Materile, H., Schoenfeld, H.-J., Stueber, D. und Takacs, B. (1991), Ability of recombinant or native proteins to protect monkeys against heterologous challenge with *Plasmodium falciparum*, Inf. Imm. 59, 3498-3503; Holder, A.A., Freeman, R.R. und Nicholls, S.C. (1988), Immunization against *Plasmodium falciparum* with recombinant polypeptides produced in *Escherichia coli*, Parasite Immunol. 10, 607-617; Herrera, S., Herrera, M.A., Perlaza, B.L., Burki, Y., Caspers, P., Döbeli, H., Rotmann, D. und Certa, U. (1990), Immunization of Aotus monkeys with *Plasmodium falciparum* blood-stage recombinant proteins, Proc. Natl. Acad. Sci. USA 87, 4017-4021; Herrera, M.A., Rosero, F., Herrera, S., Caspers, P., Rotmann, D., Sinigaglia,F. und Certa, U. (1992), Protection against malaria in Aotus monkeys immunized with a recombinant blood-stage antigen fused to a universal T-cell epitope; correlation of serum gamma interferon levels with protection, Inf. Imm. 60, 154-158; Patarroyo, M.E., Romero P., Torres, M.L., Clavijo, P., Moreno, A., Martinez, A., Rodriquez, R., Guzmann, F, und Cabezas, E. (1987), Induction of protective immunity against experimental infection with malaria using synthetic peptides, Nature 328, 629-632). In diesen Impfstudien lassen sich hierbei zwei Ansätze unterscheiden
- Verwendung von aus Parasiten isoliertem Material und
- Einsatz von in heterologen Expressionssystemen gewonnenem Material.

Letzteres bestand in der Regel aus relativ kleinen Teilbereichen des Gesamtproteins. Obwohl die ersten Resultate der in Vorversuchen an Affen durchgeführten Impfungen andeuten, daß gp190/MSP1 einen Schutz vermitteln könnte, haben alle an Primaten durchgeführten Experimente zwei Probleme, welche einen solchen Schluß in Frage stellen:
(a) sie wurden an zu kleinen Tiergruppen durchgeführt
(b) sie wurden nicht wiederholt.

Die Resultate und die daraus gezogenen Schlüsse sind daher statistisch nicht abgesichert. Neben dem schwierigen Zugang zu geeigneten Affen liegt das zugrunde liegende Hauptproblem darin, daß es bislang nicht möglich war, gutes Impfmaterial in ausreichender Menge herzustellen.

Andererseits konnten nach der Sequenzierung des gp190-Gens aus dem K1- und dem MAD20-Stamm von *Plasmodium falciparum* konnten überlappende Fragmente in *E. coli* exprimiert werden. Mit diesem Material zeigten epidemiologische Studien in Westafrika, daß in der Gruppe der Adoleszenten eine Korrelation bestand zwischen Antikörpertiter gegen gp190/MSP1-Fragmente einerseits und einem Schutz vor Parasiteninfektion andererseits. Darüber hinaus schien der Titer auch mit der Fähigkeit zu korrelieren, die Parasitämie auch auf niedrigem Niveau zu kontrollieren (Tolle et al. (1993): A prospective study of the association between the human humoral immune response to *Plasmodium falciparum* blood stage antigen gp190 and control of malarial infections. Infect Immun. 61, 40-47). Diese Resultate werden ergänzt durch neue Untersuchungen an Aotusaffen im Rahmen der vorliegenden Erfindung. Hier wurde ein hoher Schutz gegen Infektion mit dem Parasiten dadurch erreicht, daß Proteinpräparationen aus *Plasmodium falciparum*, die überwiegend aus nichtprozessiertem gp190/MSP1 bestanden, als Impfstoff benutzt worden waren. Die Affen mit dem höchsten Antikörpertiter gegen gp190/MSP1 waren am besten geschützt. Diese Resultate machten letztendlich das gp190 zu einem vielversprechenden Kandidaten für eine Impfstoff gegen Malaria tropica.

Von einigen Arbeitsgruppen wurde der C-terminalen Domäne des gp190 (p19 bzw. p42) eine besondere Rolle bei der gp190 vermittelten Immunität zugewiesen (siehe auch Chang, S.P., Case, S.E., Gosnell, W.L., Hashimoto, A., Kramer, K.J., Tam, L Q., Hashiro, C.Q., Nikaido, C.M., Gibson, H.L., Lee-Ng, C.T., Barr, P.J., Yokota, B.T. und Hui, G.S.N.(1996), A recombinant baculovirus 42-kilodalton C-terminal fragment of *Plasmodium falciparum* merozoite surface protein 1 protects Aotus monkeys against malaria, Inf. Imm. 64, 253-261; Burghaus, P.A., Wellde, B.T., Hall, T., Richards, R.L., Egan, A.F., Riley, E.M., Ripley-Ballou, W. und Holder A.A. (1996), Immunization of Aotus nancymai with recombinant C-terminus of *Plasmodium falciparum* merozoite surface protein-1 in liposomes and alum adjuvant does not induce protection against a challenge infection, Inf. Imm., in press).

Bislang ist es jedoch nicht möglich, auf rationaler Basis andere Teile des gp190 als nicht relevant für eine schützende Immunantwort auszuschließen. Es ist daher nach wie vor notwendig, das gesamte Gen bzw. das intakte gp190 für Impfversuche zu verwenden. Trotz mehrfacher Versuche verschiedener Arbeitskreise ist es jedoch noch nicht gelungen, das gesamte Gen des gp190/MSP1 zu klonieren und zu exprimieren.

Bislang war es auch noch nicht möglich, a priori einen Teil aus der gp190-Sequenz für die schützende Immunantwort als nicht relevant auszuschließen, so daß es nach wie vor notwendig ist, das gesamte Gen bzw. das gesamte Genprodukt für Impfver suche zu verwenden. Es ist jedoch trotz vieler Versuche mehrerer Arbeitskreise noch nicht gelungen, das gesamte Gen des gp190/MSP1 zu klonieren.

Es war daher eine Aufgabe der vorliegenden Erfindung, Impfmaterial in Form von vollständigem gp190/MSP1 in ausreichender Menge zur Verfügung zu stellen. Es war eine weitere Aufgabe der vorliegenden Erfindung, ein Verfahren anzugeben, mit dem dieses Impfmaterial gewonnen werden kann.

Es war außerdem eine weitere Aufgabe gemäß der vorliegenden Erfindung, eine vollständige DNA-Sequenz von gp190/MSP1 anzugeben, die in einem Wirtsorganismus exprimierbar ist.

Weiterhin war es eine Aufgabe der vorliegenden Erfindung, Wirtsorganismen anzugeben, die das vollständige Gen gp190/MSP1 enthalten.

Diese Aufgaben werden durch die in den Ansprüchen angegebenen Gegenstände gelöst.

Im folgenden werden einige Begriffe näher erläutert, um klarzustellen, wie sie in diesem Zusammenhang verstanden werden sollen.

"Rekombinantes Herstellungsverfahren" bedeutet, daß ein Protein von einer DNA-Sequenz durch einen geeigneten Wirtsorganismus exprimiert wird; wobei die DNA-Sequenz aus einer Klonierung und Fusion einzelner DNA-Abschnitte entstanden ist.

"Vollständiges gp190/MSP1-Protein" meint hier das gesamte, aus o.g. Plasmodien, insbesondere *Plasmodium falciparum*, isolierbare gp190/MSP1-Oberflächenprotein, das das Hauptoberflächenprotein der Merozoiten des o.g. Erregers darstellt sowie die Proteine mit analoger Funktion aus den anderen Plasmodiumarten, wie *P. vivax*. Der Begriff betrifft somit jeweils das Hauptoberflächenprotein der Merozoiten der 4 o.g, für den Menschen gefährlichen Malariaerreger. "Vollständiges gp190/MSP1-Gen" ist das für dieses Protein kodierende Gen. "Vollständig" bedeutet in diesem Zusammenhang, daß die gesamte Aminosäuresequenz des nativen Proteins vorhanden ist, bzw. daß die Gensequenz für die gesamte Aminosäuresequenz des nativen Proteins kodiert.

Mit umfaßt sind jedoch auch mutierte und/oder verkürzte Formen von gp190/MSP1, sofern sie das gleiche Immunisierungspotential (Impfschutz) wie das vollständige gp190/MSP1 aufweisen. Schließlich umfaßt der Begriff auch Varianten von gp190/MSP1 die sich dadurch auszeichnen, daß sie Proteinabschnitte verschiedener Allele in einem Proteinmolekül enthalten.

"FCB-1" ist ein Stamm von *P. falciparum,* wie beschrieben bei Heidrich, H.-G., Miettinen-Baumann, A., Eckerskorn, C. und Lottspeich, F. (1989) The N-terminal amino acid sequences of the Plasmodium falciparum (FCB1) merozoite surface antigens of 42 and 36 kilodalton, both derived from the 185-195-kilodalton precursor. Mol. Biochem. Parasitol. 34, 147-154.

"Ankersignal" meint hier eine Proteinstruktur, für die eine DNA-Sequenz am 3'- oder 5'-Ende des erfindungsgemäßen Gens kodiert. Ankersignale sind Strukuren, die einem Polypeptid die Verankerung an anderen Strukturen, wie z.B. Membranen ermöglichen.

"Signalpeptid" bedeutet hier eine Proteinstruktur, für die eine DNA-Sequenz am N-terminalen Ende des erfindungsgemäßen Gens kodiert. Signalpeptide sind Strukturen, die dem Polypeptid u.a. ein Einschleusen in Membranen ermöglichen.

"AT-Gehalt" meint im Zusammenhang der vorliegenden Erfindung die prozentuale Menge von Adenin/Thymin-Basenpaaren im Verhältnis zu Guanin/Cytosin-Basenpaaren.

"Klonierung" soll hier alle im Stand der Technik bekannten Klonierungsmethoden umfassen, die hier zum Einsatz kommen könnten, die jedoch nicht alle im einzelnen beschrieben werden, weil sie zum selbstverständlichen Handwerkszeug des Fachmanns gehören.

"Expression in einem geeigneten Expressionssystem" soll hier alle im Stand der Technik bekannten Expressionsmethoden in bekannten Expressionssystemen umfassen, die hier zum Einsatz kommen könnten, die jedoch nicht alle im einzelnen beschrieben werden, weil sie zum selbstverständlichen Handwerkszeug des Fachmanns gehören.

Es ist eine erste Aufgabe gemäß der vorliegenden Erfindung, ein Verfahren anzugeben, durch das das gp190/MSP1-Protein und das Gen hierfür in ausreichender Menge ohne übermäßige Kosten produziert werden kann.

Diese Aufgabe wird durch das in Anspruch 1 beschriebene rekombinante Herstellungsverfahren gelöst, durch das ein voll-ständiges gp190/MSP-1-Gen und das davon kodierte Protein in ausreichender Menge erhältlich ist.

Durch dieses Verfahren wurde es erstmals möglich, das Protein in seiner Gesamtheit außerhalb des Parasiten zu synthetisieren. Das so synthetisierte Protein ist, wie die Analyse mit konformationelle Epitope erkennenden monoklonalen Antikörpern zeigt, zumindest über weite Bereiche in natürlich gefalteter Form herstellbar. Durch das rekombinante Herstellungsverfahren konnten jeweils mehrere Milligramm intaktes gp190/MSP1 aus den Wirtsorganismen gewonnen werden, eine Menge, die aus technischen und aus Kostengründen aus Parasiten praktisch nicht gewonnen werden kann. Die jetzt mögliche Produktion des Proteins in beliebigen Mengen eröffnet neue Perspektiven für seinen Einsatz als experimentellen Impfstoff gegen Malaria. Darüber hinaus ist der Weg frei für die Entwicklung von Lebendimpfstoffen sowie für Vakzine auf Nukleinsäure-Basis.

Vorzugsweise liegt der Synthese der für das Protein gp190/MSP1 kodierenden GenSequenz die Sequenz des *P. falciparum* FCB-1 Stammes zugrunde. *P*. *falciparum* ist der Erreger der Malaria tropica und damit der gefährlichste unter den Malaria-Arten. Das zugrunde liegende Gen ist ein Vertreter des "K1-Allels", wobei K1 für einen bestimmten *P. falciparum*-Stamm steht. Seine kodierende Sequenz erstreckt sich über 4917 Basenpaare und schließt eine Signalsequenz am N-terminalen Ende sowie eine Anker-Sequenz am C-terminalen Ende ein.

Weiterhin ist das rekombinante Herstellungsverfahren gemäß der Erfindung vorzugsweise dadurch gekennzeichnet, daß der AT-Gehalt der dem Protein zugrunde liegenden DNA-Sequenz gegenüber der Wildtyp-Sequenz erniedrigt ist, von 74% im ursprünglichen Gen auf vorzugsweise ca. 55%, indem bspw. unter Erhalt der Aminosäuresequenz des FCB-1-Proteins eine DNA-Sequenz mit den im menschlichen Genom üblichen Codon-Häufigkeiten hergestellt wird. Andere Codonhäufigkeiten, welche den AT-Gehalt emiedrigen, sind ebenfalls denkbar.

Vorzugsweise kodiert das dem durch das rekombinante Herstellungsverfahren erzeugte Protein zugrunde liegende Gen für die volle Aminosäuresequenz einschließlich Signalpeptid und GPI-Ankersignalpeptid, im weiteren als gp190^{S} bezeichnet.

In einer weiteren bevorzugten Ausführungform kodiert das dem durch das rekombinante Herstellungsverfahren erzeugte Protein zugrunde liegende Gen für die volle Aminosäuresequenz mit Ausnahme des GPI-Ankersignals. Diese Ausführungsform wird im weiteren als gp190^{S1} bezeichnet.

In noch einer weiteren bevorzugten Ausführung kodiert das dem durch das rekombinante Herstellungsverfahren erzeugte Protein zugrunde liegende Gen für die volle Aminosäuresequenz mit Ausnahme des GPI-Ankersignals und des Signalpeptids. Diese Ausführungsform wird im weiteren als gp190^{S2} bezeichnet.

In einer weiteren bevorzugten Ausführungsform kodiert das dem durch das rekombinante Herstellungsverfahren erzeugte Protein zugrunde liegende Gen für die volle Aminosäuresequenz und eine Transmembranankersequenz.

In einer besonders bevorzugten Ausführungsform umfaßt das rekombinante Herstellungsverfahren folgende Schritte.

Zunächst den Entwurf der zu synthetisierenden DNA-Sequenz auf der Basis des Gens aus *P. falciparum* FCB-1, wobei eine DNA-Sequenz mit den z.B. im menschlichen Genom üblichen Codon-Häufigkeiten unter Beibehalten der Aminosäuresequenz des FCB-1-Proteins hergestellt wird.

Hierdurch sollte der AT-Gehalt des Gens reduziert werden, vorzugsweise auf 55%.

Im weiteren Verfahren wird die entworfene Sequenz bspw. in 5 überlappende Regionen eingeteilt, welche jeweils Domänen der natürlichen Prozessierungsprodukte des gp190/MSP1-Proteins aus FCB-1 entsprechen: p83, p31, p36, p30 und p19.

Es werden Desoxyoligonukleotide synthetisiert, die jeweils mindestens die gesamte Länge einer Region abdecken.

Besonders bevorzugt werden die Desoxyoligonukleotide so synthetisiert, daß ihre Sequenz abwechselnd dem "oberen" (5' -3') bzw. dem "unteren" (3' - 5') DNA-Strang entspricht. Die Länge dieser Oligonukleotide ist vorzugsweise durchschnittlich 120 Nukleotide und sie überlappt die benachbarten Sequenzen jeweils um ca. 20 Basen.

In einer möglichen Ausführungsform werden DNA-Sequenzen von etwa doppelter Länge wie die jeweiligen Ausgangsproduke durch asymmetrische PCR hergestellt und zwar so, daß die überschüssigen DNA-Sequenzen, die benachbart sind, jeweils den gegenüberliegenden Strang repräsentieren. Dies führt in einem zweiten

PCR-Amplifikationszyklus zu einem Zweitprodukt, das der Länge von vier ursprünglich eingesetzten Oligonukleotiden (abzüglich der überlapppenden Region) entspricht. Die Überführung dieser Produkte in ein überwiegend aus Einzelstrang-DNA bestehendes Präparat durch asymmetrische PCR mit den endständigen Oligonukleotiden erlaubt in einem weiteren Amplifikationsschritt die Herstellung eines 800 bp langen doppelsträngigen DNA-Fragments in nur 25 PCR-Zyklen.

Auf diese Weise werden direkt die kodierenden Regionen für p19, p30, p36 und p31 synthetisiert und in *E. coli* molekular kloniert. Klone mit fehlerfreier Sequenz wurden entweder direkt oder durch Zusammensetzen fehlerfreier Teilsequenzen erhalten. Die Region, welche das p83 kodiert, wurde durch Fusion aus zwei etwa 1200 bp umfassenden Sequenzen erhalten.

Im weiteren Verlauf des Verfahrens wurden die einzelnen Sequenzen kloniert. Als Expressionsvektoren bieten sich vorzugsweise die Plasmide pDS56, RBSII ("Hochuli, E., Bannwarth, W. Döbeli, H. Gentz, R., and Stüber, D. (1988) Genetic approach to facilitate purification of recombinant proteins with a novel metal chelate adsorbent. Biotechn. 6, 1321-1325"), pBi-5 ("Baron, U., Freundlieb, S., Gossen, M. and Bujard, H. (1995) Corregulation of two gene activities by tetracycline via a bidirectional promoter. Nucl. Acids Res. 23, 3605-3606") und ppTMCS an. Es sind jedoch auch andere Expressionsvektoren denkbar.

Bevorzugte Wirtsorganismen für die Expression sind *E. coli*, besonders bevorzugt der Stamm DH5alphaZ1 (R. Rutz, Dissertation 1996, Universität Heidelberg), HeLa-Zellen, CHO-Zellen, Toxoplasma gondii (Pfefferkorn, E.R. and Pfefferkorn, C.C. 1976, Toxoplasma gondii: Isolation and preliminary characterization of temperature - sensitive mutants. Exp. Parasitol. 39, 365-376) oder Leishmania. Weitere Wirtssysteme könnten z.B. Hefen, Baculoviren oder Adenoviren sein, wobei der Gegenstand der Erfindung nicht auf die genannten Wirtssysteme beschränkt sein sollte.

Es war eine weitere Aufgabe der vorliegenden Erfindung, eine vollständige, zur Expression geeignete DNA-Sequenz des gp190/MSP1-Oberflächenproteins von *P*. *falciparum* anzugeben.

Diese Aufgabe wird durch die in Anspruch 17 genannte Erfindung gelöst, wobei die Sequenz durch das oben beschriebene rekombinante Herstellungsverfahren erhältlich ist.

In einer bevorzugten Ausführungsform der vorliegenden Erfindung kodiert die zur Expression geeignete DNA-Sequenz für die vollständige Aminosäuresequenz.

In einer anderen bevorzugten Ausführungsform der vorliegenden Erfindung kodiert die zur Expression geeignete DNA-Sequenz für die vollständige Aminosäuresequenz mit Ausnahme des Ankersignals.

In einer weiteren bevorzugten Ausführungsform gemäß der vorliegenden Erfindung kodiert die zur Expression geeignete DNA-Sequenz für die vollständige Aminosäuresequenz mit Ausnahme des Ankersignals und des Signalpeptids. Diese Ausführungsform von gp190/MSP1 kann dadurch gekennzeichnet sein, daß sie am N-Terminus 1.1 zusätzliche Aminosäuren, davon 6 Histidine, enthält.

Besonders bevorzugt enthält die zur Expression geeignete DNA-Sequenz keine erkennbaren "splice-donor" und "splice-acceptor"-Signale, und sie ist vorzugsweise dadurch gekennzeichnet, daß sie keine größeren GC-reichen Sequenzen enthält, die stabile Haamadelstrukturen auf RNA-Ebene bewirken könnten.

Vorzugsweise sollten Erkennungssignale für Restriktionsenzyme, welche Sequenzen von sechs und mehr Basenpaaren erkennen, vermieden werden.

In einer bevorzugten Ausführung werden spezifische, d.h. nur einmal im Gen vorkommende Schnittstellen für Restriktionsendonukleasen in Regionen eingeführt, welche die nach der Prozessierung des Proteins entstehenden Domänen trennen.

Besonders bevorzugt sollten an beiden Enden des Gens Sequenzen für Restriktionsendonukleasen vorhanden sein, die im Gen nicht vorkommen.

Weiterhin werden durch die Erfindung Wirtsorganismen zur Verfügung gestellt, die die vollständige Nukleinsäntesequenz des gp190/MSP-1 Oberflächenproteins gemäß einem der Ansprüche 17 - 25 enthalten.

Solche Wirtsorganismen sind vorzugsweise *E. coli,* besonders bevorzugt der Stamm DH5alphaZ1, HeLA-Zellen, CHO-Zellen, Toxoplasma gondii oder Leishmania. Die HeLa-Zellen und CHO-Zellen sollten vorzugsweise konstitutiv tTA synthetisieren.

Schließlich stellt die vorliegende Erfindung eine Möglichkeit zur Verfügung, ein nach dem rekombinanten Herstellungsverfahren erzeugtes gp190/MSP1-Oberflächenprotein oder Teile desselben zur aktiven Immunisierung gegen Malaria zu verwenden.

Das hier vorgestellte Syntheseschema erlaubt auch das zweite Allel des gp190/MSP1-Gens herzustellen. Damit wird dem Dimorphismus des Proteins Rechnung getragen. Die Hauptvariabilität des Proteins beruht jedoch auf den Sequenzen von zwei relativ kurzen Blöcken (Block II und IV, Ref. 1), die oligomorph sind. Die vorliegenden Sequenzdaten ermöglichen es, daß mit 6-8 Sequenzkombinationen dieser Blöcke über 95% aller bekannten gp190/MSP1-Sequenzen abgedeckt werden können. Die Synthese dieser Sequenzvarianten läßt sich problemlos anhand der hier vorgestellten Strategien verwirklichen, so daß Varianten sowohl in das K1 als auch in das MAD20-Allel eingebaut werden können. Impfstoffe aus den hierdurch entstehenden Sequenzfamilien können ggf. gegen ein breites Spektrum von Parasiten mit gp190/MSP1-Varianten Schutz verleihen.

Die Herstellung von verschiedenen Impfstofftypen ist möglich:
- Auf der Ebene von Proteinpräparaten, wobei jeweils Mischungen der zwei Familien (K1-Typ, MAD20-Typ mit verschiedenen Varianten der Blöcke II und IV) zur Anwendung kommen können. Verschiedene Träger bzw. Adjuvans-Materialien können zum Einsatz kommen: Aluminiumoxid, Liposomen, lscoms QSz1 etc.
- Auf der Ebene der Lebendimpfstoffe: (a) virale Träger, insbesondere Vakzinia und Adenoviren; (b) Parasiten als Träger, insbesonder avirulente Formen von Leishmania und Toxoplasma; (c) bakterielle Träger, z.B. Salmonella
- Auf der Ebene der Nukleinsäuren, wobei bspw. für die Gentherapie geeignete Vektoren als Vehikel zum Einbringen der Gene in den Wirt verwendet werden; weiterhin ist das Einbringen von Ribonukleinsäuren, welche das gewünschte Protein kodieren, denkbar.

Eine weitere Möglichkeit der Impfung besteht in der Verwendung eines gemäß dem erfindungsgemäßen rekombinanten Herstellungsverfahren erzeugten gp190/MSP1-Proteins zur Herstellung von monoklonalen Antikörpern, die dann ihrerseits zur passiven Immunisierung gegen Malaria verwendet werden.

Ebenso wird eine Verwendung der gemäß dem rekombinanten Herstellungsverfahren in einem Zwischenschritt entstandenen, dem Protein zugrunde liegenden DNA-Sequenz zur Herstellung einer Vakzine auf Nukleinsäurebasis ermöglicht.

Weiterhin wird durch die Erfindung ein stabilisiertes. Gen für das gp190/MSP-1 Oberflächenprotein gemäß der vorliegenden Erfindung zur Verfügung gestellt, das sich dadurch auszeichnet, daß es einen geringeren AT-Gehalt aufweist als das Wildtype-Gen.

Im folgenden soll die Erfindung anhand der Abbildungen und Tabellen sowie einiger Beispiele in einzelnen Ausführungsformen beschrieben werden.

Dabei zeigt
Abb. 1: Schematische Darstellung des gp190/MSP1 Vorläuferproteins aus *P. falciparum* (FCB-1).
Abb. 2: Zwei mit nativem gp190/MSP1 aus *P. falciparum* (FCB-1) an Aotus-Affen durchgeführte Impfversuche.
Abb. 2A: mit 3 x 60 Mikrogramm gp190/MSP1
Abb. 2B: mit 3 x 40 Mikrogramm gp190/MSP1
Abb. 3A: Strategie der Synthese des gp190/MSP1-Gens
Abb. 3B: Prinzip der PCR-gestützten Total-Synthese
Abb. 3C: Totalsequenz des gp190^{S}
Abb. 3D: N- und C-Terminus der gp190^{S1}-Variante
Abb. 4A: Expressionsvektor pDS56 mit gp190^{S2}-Sequenz
Abb. 4B: Gelelektrophorese von gp190^{S2}.
Abb. 5A: Expressionsvektor pBi-5 mit gp190^{S1}-Sequenz
Abb. 5B: Immunfluoreszenz von HeLa-Zellen
Abb. 5C: Elektrophoretische Charakterisierung von aus HeLa-Zellen aufgereinigtem gp190^{S1}
Abb. 6A: Expressionsvektor ppT 190 mit gp190-Sequenz
Abb. 6B: Immunfluoreszenz der Expression von gp190^{S} in *T*. *gondii*
Abb. 6C: Polyacrylamid-Gelelektrophorese von gp190 aus *T. gondii*

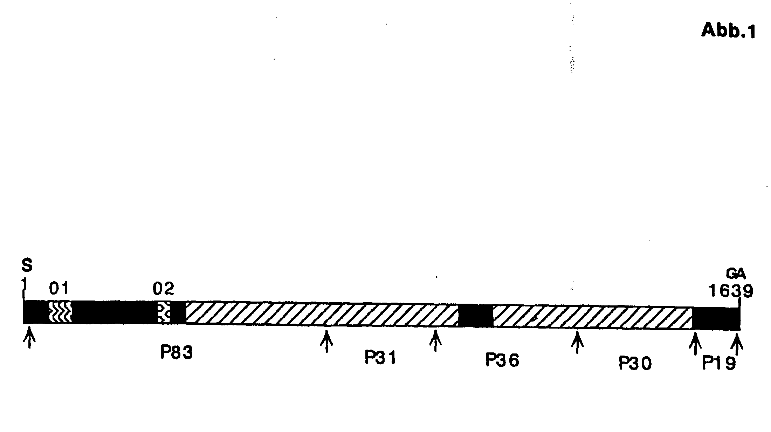

Bei dem in Abb. 1 schematisch dargestellten gp190/MSP1-Vorläuferprotein aus *P*. *falciparum* (FCB-1) repräsentieren die dunklen Blöcke Regionen, die in allen Stämmen hochkonserviert vorliegen. Die schraffierten Blöcke zeigen die dimorphen Bereiche, welche im Falle des FCB-1 lsolates dem K1-Allel entstammen. O1 und O2 zeigen die oligomorphen Bereiche. S bezeichnet die Signalpeptidsequenz, welche 19 Aminosäuren umfaßt, GA, die C-terminale Region, welche das Signal für die GPI-Verankerung des Proteins in der Membran enthält. Die Pfeile deuten die Prozessierungsstellen an, durch die Proteine p53, p31, p36, p30 und p19 entstehen. Das gp190-Gen kodiert insgesamt 1639 Aminosäuren.

Die weiteren Abbildungen werden im Zusammenhang mit den folgenden Beispielen ausführlicher erläutert.

### BEISPIELE

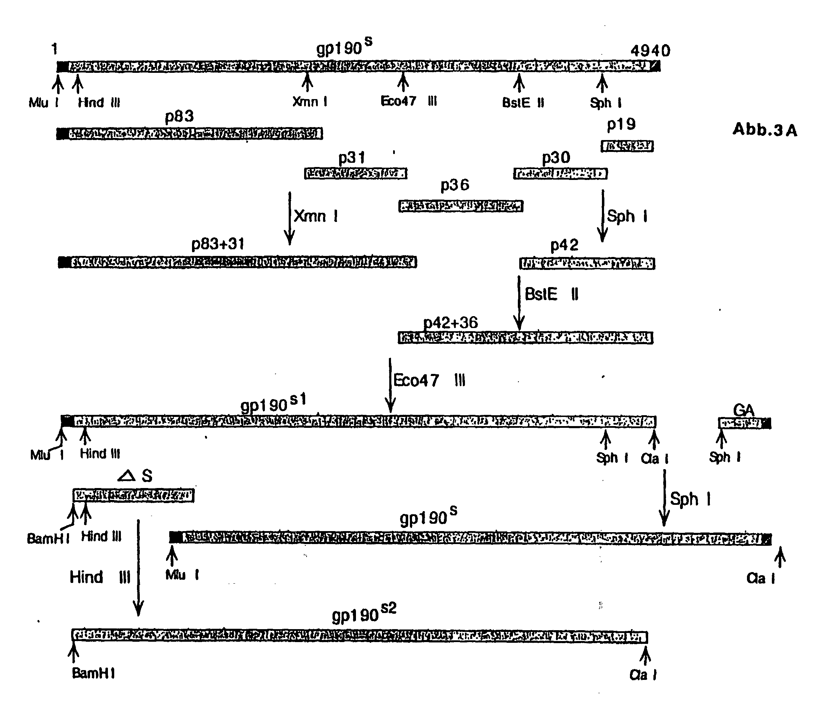

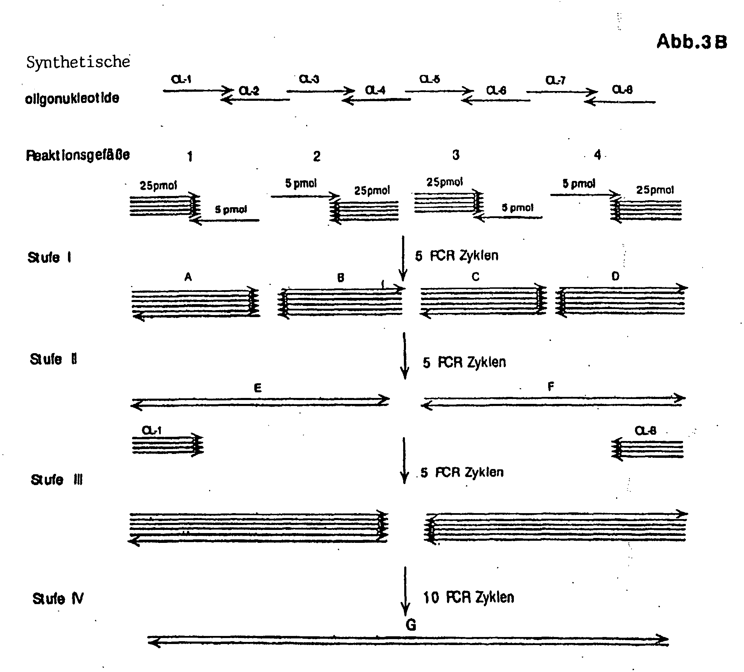

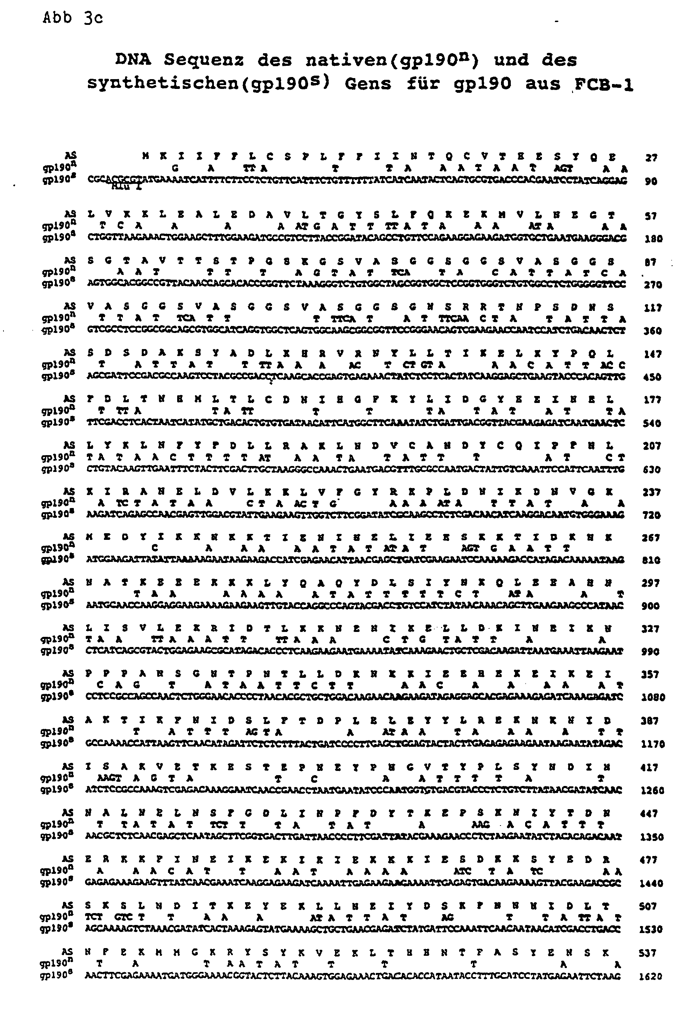

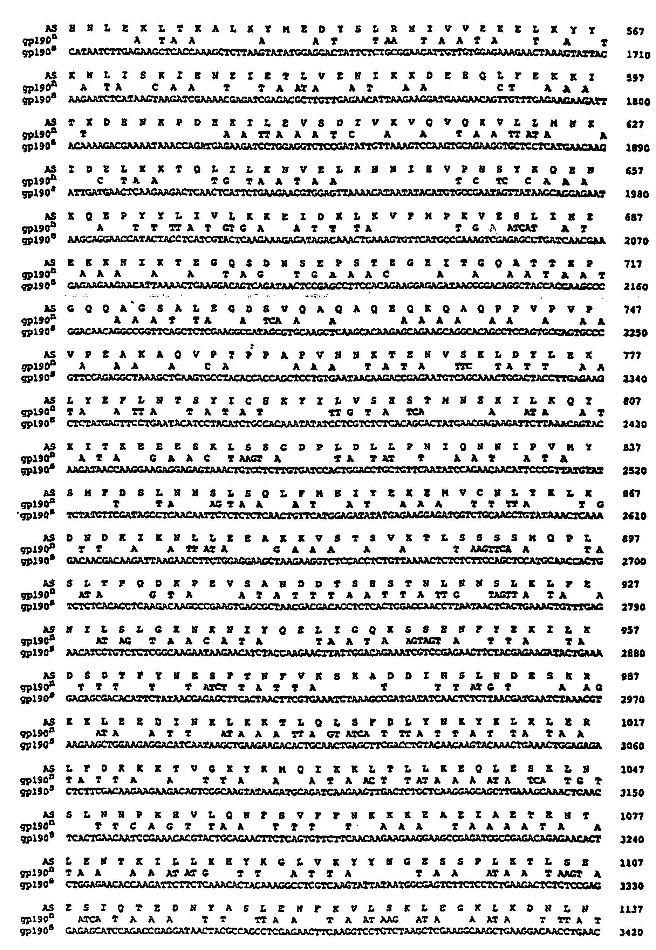

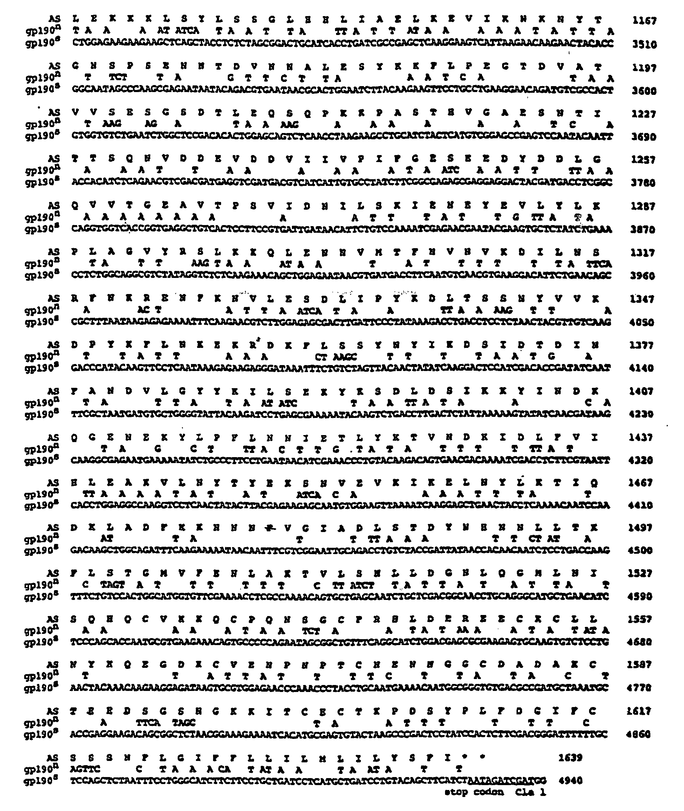

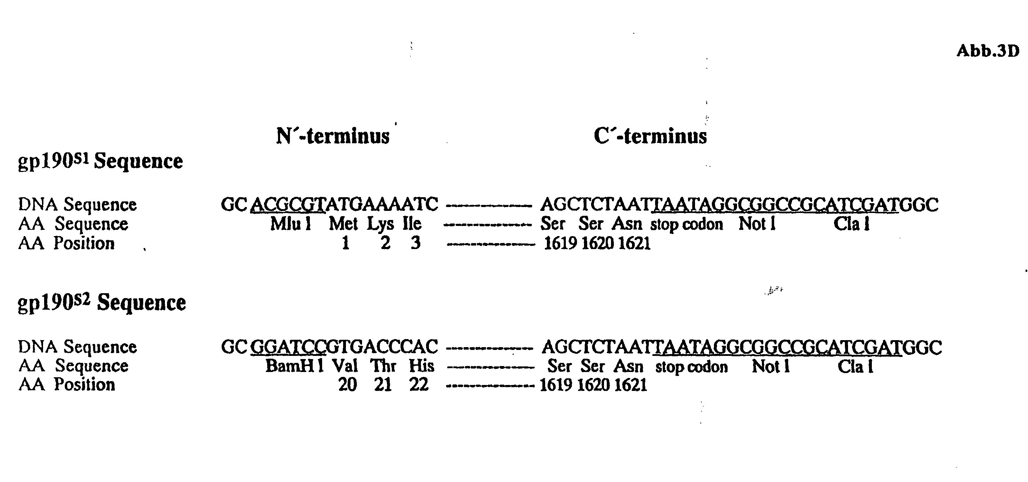

### Beispiel 1: Totalsynthese einer das gp190/MSP1 kodierenden DNA-Sequenz (siehe hierzu Abb. 3)

### A. Strategie der Synthese des gp190/MSP1-Gens (gp190^{S})(siehe Abb. 3A).

Die Sequenz wurde aufgeteilt in Fragemente, die den Hauptprozessierungsprodukten entsprachen: p83, p31, p36, p30 und p19. In den Übergangsregionen wurden Spaltstellen für Restriktionsendonukleasen (Pfeile in Abb. 3) so eingeplant, daß die Aminosäuresequenz nicht verändert wurde. Alle hier aufgeführten Schnittstellen kommen in der Sequenz nur einmal vor.

Die Fragmente wurden überlappend synthetisiert, so daß die Schnittstellen an den jeweiligen Enden die Verknüpfung zu den benachbarten Fragmenten durch Ligierung möglich machten. Alle Einzelfragmente enthielten zusätzlich an ihrem 5'-Ende eine BamHI-Schnittstelle zur Insertion in Expressionsvektoren. Über MluI und ClaI konnte die Gesamtsequenz kloniert werden. Das hier gezeigte Schema führt zunächst zu einer Sequenz, welche den GPI-Anker nicht ausbilden kann, da C-terminal 18 Aminosäuren fehlen. Die Synthese eines entsprechenden Oligonukleotids, sowie eines über die Sphl-Schnittstelle sich erstreckenden "Primers" führt nach PCR zu dem Fragment GA, das über Sphl und Clal eingesetzt werden konnte, die resultierende Totalsequenz war gp190^{S}. Zur Entfernung der das Signalpeptid kodierenden Sequenz wurden "PCR-Primer" hergestellt, über die das Fragment ΔS synthetisiert wurde. Es erlaubt, über eine BamHI und eine HindIII-Schnittstelle den N-Terminus so zu verändern, daß das Protein mit Aminosäure Nr. 20 begann. Die Kernsequenz, welche das gp190/MSP1 ohne Signalsequenz und ohne GPI-Anker-Signal kodiert, wurde mit gp 190^{S2} bezeichnet. Deletion des GPI-Ankersignals allein führte zu gp190^{S1}.

### B. Prinzip der PCR-gestützten Totalsynthese (siehe Abb. 3B)

Oligodesoxynukleotide von etwa 120 Nukleotiden Länge wurden abwechselnd vom kodierenden bzw. nichtkodierenden Strang so synthetisiert, daß sie jeweils etwa 20 Basen mit dem benachbarten Fragement überlappten. Das Schema zeigt beispielhaft die Synthese eines ca. 800 bp langen Fragments aus Oligonukleotiden. In der ersten Stufe wurden in 4 Reaktionsgefäßen jeweils 2 Oligonukleotide "asymmetrisch" amplifiziert. Es entstanden 4 etwa 220 bp lange DNA-Populationen, die vorwiegend aus Einzelsträngen bestanden (A, B, C, D). Vereinigung von A und B sowie von C und D und Amplifikation über 5 Zyklen führte zu 2 etwa 400 bp langen doppelsträngigen Produkten. Asymmetrische Amplifikation dieser DNA-Fragmente (Stufe III) ergab Einzelstrangpopulationen, welche nach Vereinigung und Amplifikation (Stufe IV) nach 10 Zyklen das Endprodukt G von ca. 800 bp Länge ergaben. Diese Synthese war ohne Isolierung der Zwischenprodukte und ohne Puffer oder Enzymemeuerung durchführbar, und war nach 3 Stunden beendet. Das Endprodukt wurde elektrophoretisch gereinigt, mit den geeigneten Restriktionsendonukleasen nachgeschnitten und im pBluescript (Stratagene), in dessen Polylinker eine Mlul und eine Clal-Schnittstelle eingesetzt worden waren, in *E. coli* kloniert.

### C. Totalsequenz des gp190^{S} (siehe Abb. 3C)

Nach Fusion aller Teilsequenzen (Abb. 3A) in pBluescript wurde die Sequenz des Gens mit der Dideoxymethode verifiziert. Das Leseraster des gp190^{S} hatte eine Länge von 4917 bp (+2 Stopcodons) und kodierte eine Aminosäuresequenz, welche der des gp190/MSP1 aus FCB-1 entspricht (1639 Aminosäuren).

### D. N- und C-Terminus der gp190^{S1}-Variante (siehe Abb. 3D)

Die N-terminale Sequenz, beginnend mit der BamHI-Schnittstelle, zeigte den Übergang bei Aminosäure 20, von der angenommen wird, daß sie nach Abspaltung des Signalpeptids den N-Terminus definiert. Am C-Terminus war die kodierte Sequenz bei Aminosäure 1621 zu Ende. Den Stopcodons folgte die Clal-Schnittstelle.

### Beispiel 2: Expression des gp190^{S2} in E. coli

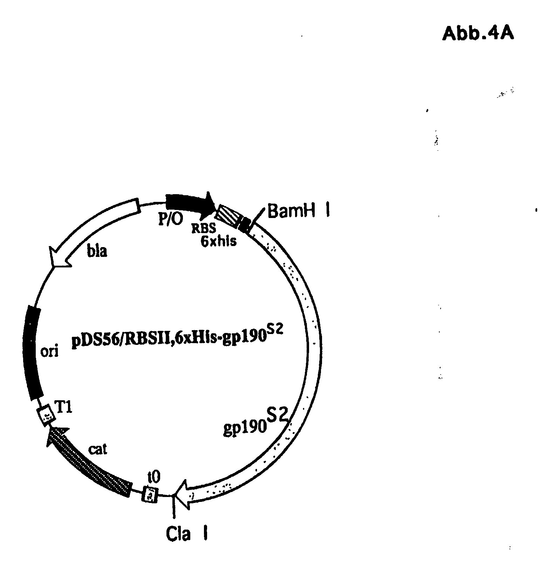

### A. Expressionsvektor (siehe Abb. 4A)

Die gp190^{S2}-Sequenz wurde über die BamHI- und ClaI-Schnittstellen in pDS56RBSII eingesetzt. Dadurch wurden 6 Histidine sowie einige aus dem Vektor stammenden Aminosäuren an den N-Terminus fusioniert; dies ergibt folgende N-terminale Sequenz des Leserasters; Met Arg Gly Ser (His)₆ Gly Ser. Durch den Promotor P_{N25lacO-1} stand die Transkription unter lacR/O/IPTG-Kontrolle.

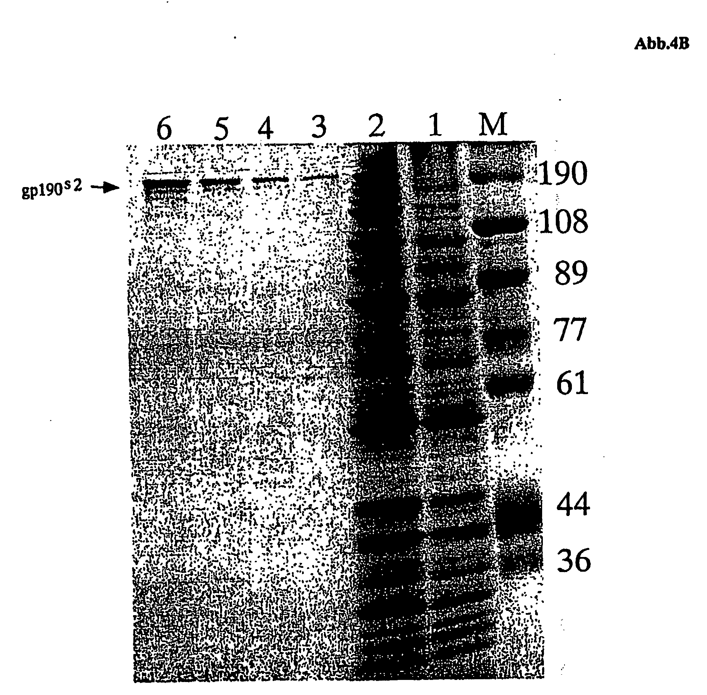

### B. Expression und Aufreinigung von gp190^{S2} (siehe Abb. 4B)

Eine Überführung des Vektors pDS56RBSIIgp190^{S2} in *E*. *coli* DH5αZ1 und Induktion der Synthese durch IPTG ergab nach elektrophoretischer Auftrennung des Protein-Totalextraktes der Kultur eine deutlich sichtbare Bande in der erwarteten Größe (Pfeil). Die Aufreinigung des Materials durch IMAC und Affinitätschromatographie (Antikörpersäule mit mAK5.2) führte zu einem homogenen Produkt von etwa 190 kD. In der Abb. bedeuten M= Motekulargewichtsstandards; 1= *E. coli* Proteine vor, 2= nach Induktion mit IPTG für 2 Stunden. 3,4,5 = Fraktionen aus der Elution der mAK-Säule.

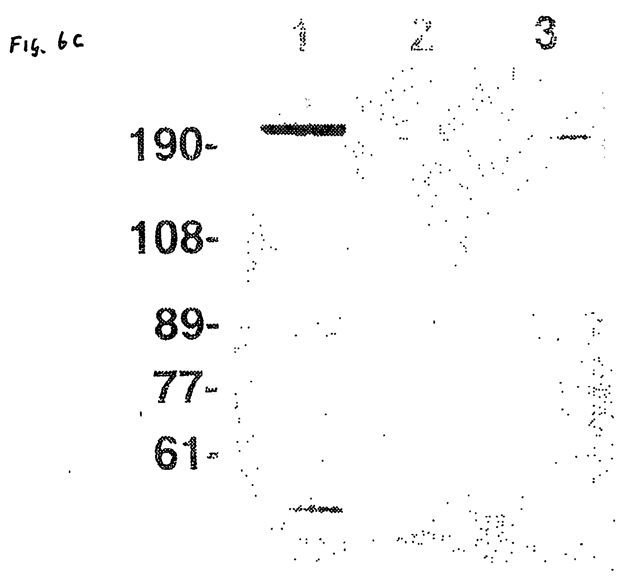

### Beispiel 3: Tetrazyklin-kontrollierte Expression von gp190^{S1} in HeLa- und CHO-Zellen und Isolation des Produktes (siehe auch Abb. 51 bzw. 6c)

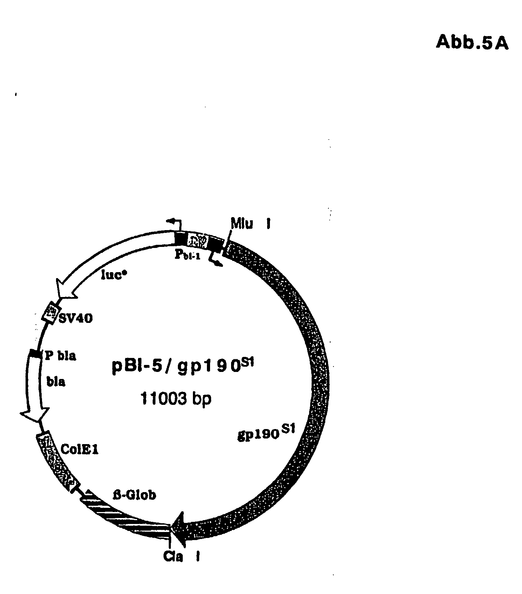

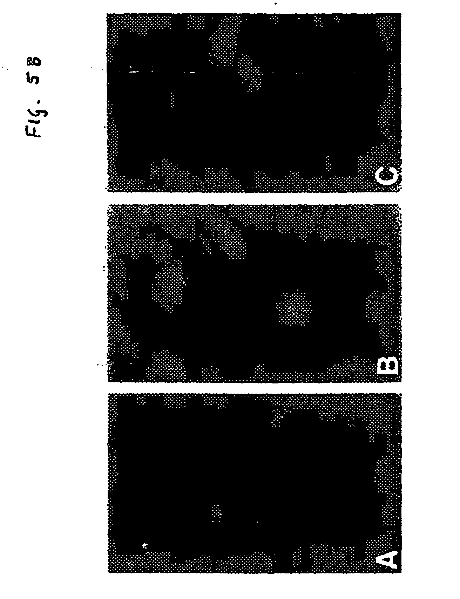

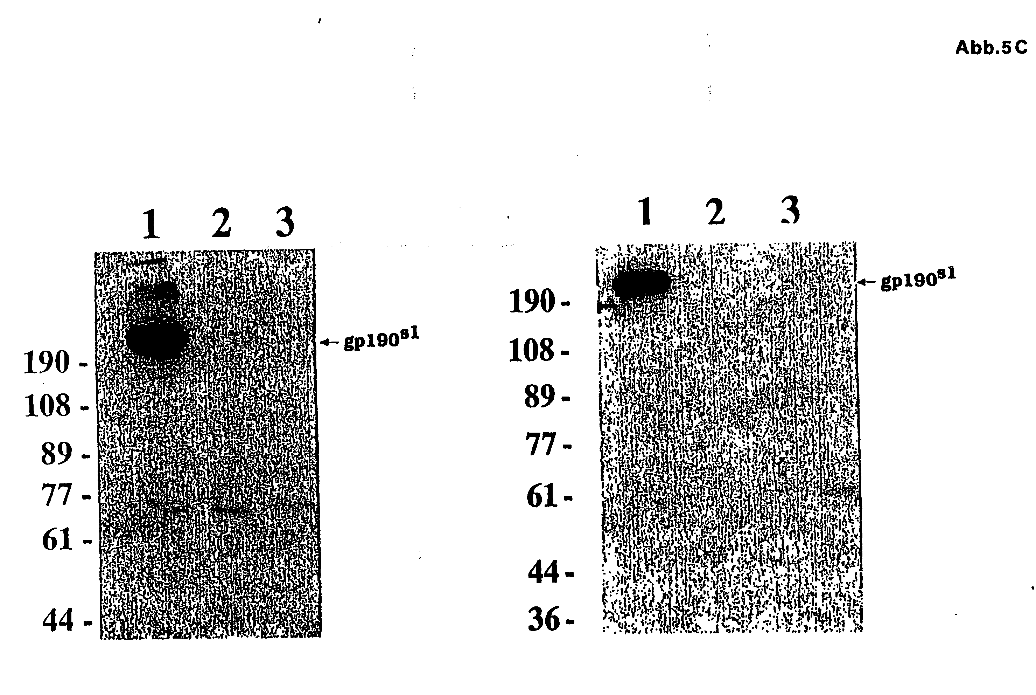

A. Die gp190-Sequenz wurde in den Expressionsvektor pBi-5 über die BamHI/ClaI-Schnittstellen eingesetzt. Damit stand die Transkription des Gens unter Kontrolle eines bidirektionalen "tTA-responsive"-Promotors und konnte über Tc reguliert werden. Der bidirektionale Promotor initiierte gleichzeitig die Transkription des Indikatorgens Luciferase. Damit ließ sich die Regulation der Expression leicht verfolgen (siehe auch Abb. 5A).
B. Immunfluoreszenz von HeLa-Zellen, welche Luciferase und gp190^{S1} Tckontrolliert exprimieren.
   In HtTA93-9-Zellen, welche die bidirektionale Transkriptionseinheit von (A) enthalten, wurde mit Antikörpern die Produktion von Luciferase (links), gp190^{S1} (Mitte) in Abwesenheit von Tc nachgewiesen. Nach Zugabe von Tc zeigte sich keine nennenswerte Synthese von gp190^{S1}, (wie dargestellt in Abb 5B, rechts).
C. Elektrophoretische Charkaterisierung von aus HeLa-Zellen aufgereinigtem gp190^{S1}.
   HeLa-Zellklon HtTA93-9 sowie CHO-Zellklon CHO27-29 wurden mit oder ohne Tc kultiviert. Durch Elektrophorese aufgetrennte Zellextrakte wurden mittels "Western blot" mit mAK5.2 analysiert (Abb. 5C); links zeigt eine Analyse der CHO-, rechts der HeLa-Zellinie. (1) = Kultur ohne, (2) = Kultur mit Tc, (3) = nicht transfizierte HtTA-1-Zellinie, Molekulargewichtsstandards sind jeweils links angedeutet.
D. Aufreinigung von in Hela-Zellklon HtTA93-9 synthetisiertem gp190^{S1}
   Die präparative Aufzucht der HtTA-93-9-Linie und Induktion der Expression von der gp190^{S1} durch Tc-Entzug erlaubte die Isolierung des Genproduktes über Affinitätschromatographie (mAK5.2-Säule).
   Das Coomassie gefärbte Polyacrylamid-Gel (Abb. 6C) zeigte nach Elektrophorese ein Produkt, das aus gp190^{S1} sowie einem weiteren Protein von ca 50 kD bestand. Letzteres war kein Derivat von gp190^{S1}, stammte also aus HeLA-Zellen. Seine gezielte Abtrennung sollte jedoch keine prinzipielle Schwierigkeit darstellen.

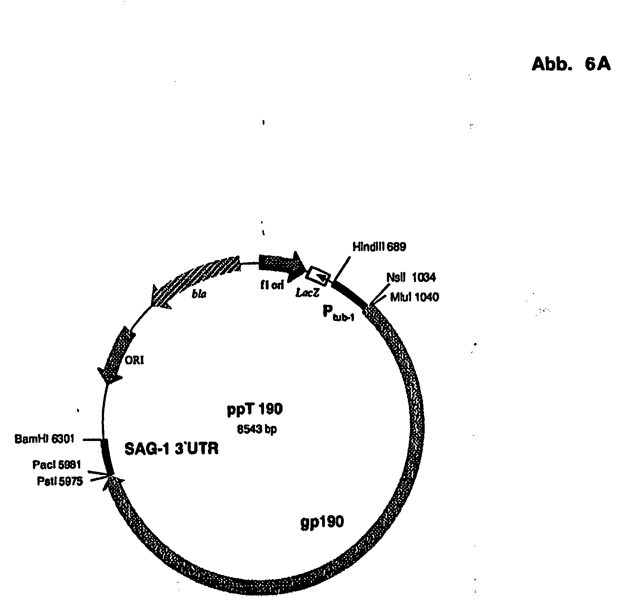

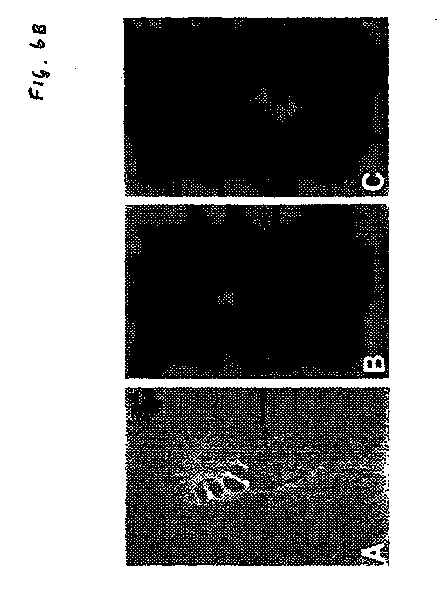

### Beispiel 4: Expression von gp190^{S} in Toxoplasma gondii und Aufreinigung des Produktes (siehe hierzu auch Abb. 6).

A. Die gp190^{S}-Sequenz wurde über MluI/PstI in den Vektor ppT eingesetzt. Damit wurde das Gen unter die Kontrolle des Tubulin-Promotors (P_{tub-1}) von T. gondii gebracht. Die 3' nicht-translatierte Region (VTR) stammte von dem Hauptoberflächenprotein von T. gondii (SAG-1) ab.
B. Expression von gp190^{S} in T. gondii.
   Transfektion von T. gondii mit pTT190 führte zur Isolierung von Parasitenlinien, die konstitutiv gp190^{S} exprimierten. Die Immunfluoreszenz mit mAK5.2 (mittleres Bild) zeigte nicht nur die Expression des Gens, sondern legte auch die Verankerung des Expressionsproduktes an der Oberfläche des Parasiten nahe, da es, wie SAG-1, das gleiche Immunfluoreszenz-Muster erzeugte (rechter Teil der Abb. 68); links in Abb. 6B ist eine Phasenkontrastaufnahme des mittleren Bildes dargestellt.
C. Isolierung von gp190^{S} aus T. gondii.
   Aus präparativen Mengen von T. gondii (5x10⁹ Parasiten) wurde gp190^{S} mittels Affinitätschromatographie (mAK5.2-Säule) aufgereinigt. Das hochreine Protein besaß das erwartete Molekulargewicht, wie das Coomassie-gefärbte Polyacrylamid-Gel nach Elektrophorese zeigte (2-3 aus Abb. 6C). Bei Nr. (1) Abb. 6C ist gereinigtes gp190^{S1} aus CHO-Zellen dargestellt mit Molekulargewichtsmarkierung an der linken Seite.

### Beispiel 5: Charakterisierung des gp190^{S} mit monoklonalen Antikörpern.

Die Wechselwirkung von 16 monoklonalen Antikörpern mit gp190^{S} aus den verschiedenen heterologen Expressionssystemen wurde durch Immunfluoreszenz (IFA) an *P. falciparum* und T. gondii bzw. durch "Western blot" an den aufgereinigten Proteinen überprüft. Völlige Übereinstimmung wurde gefunden, wenn die beiden Parasiten verglichen wurden (Zahl der + deutet die relative Intensität der Fluoreszenz an). Im Western blot reagieren12mAK's mit gp190^{S} aus *E. coli* und T. gondii. Im Gegensatz dazu binden 3 Antikörper nicht an das aus CHO-Zellen isolierte Material. Antikörper 15 und 16, die Epitope aus dem oligomorphen bzw. dem alternativen Allel (MAD20) erkennen, reagieren nicht. Die Ergebnisse sind in Tab. 1 zusammengefaßt, wobei ND = nicht durchgeführt bedeutet.

### Beispiel 6: Expression des gp190^{S} in heterologen Systemen.

### 1. Expression in E. coli

Das gp190^{S2} wurde in den Expressionsvektor, pDS56, RBSII eingesetzt, wo es unter Kontrolle des P_{N25lacO-1}-Promotors stand, der über das lac Operator/Repressor/IPTG-System regulierbar ist (Abb. 4A). Die Überführung des Plasmids in Repressor-produzierende *E. coli*-Zellen, z.B. *E*. *coli* DH5αZ1 erlaubt es, das gp190^{S2} unter IPTG-Kontrolle zu exprimieren. Das Produkt war aus dem Rohextrakt mittels einer Nickel-Chelatsäule über die durch den Vektor eingebrachte N-terminale (His)₆-Sequenz isolierbar. Eine anschließende Affinitätschromatographie an einer Antikörpersäule führte zu einem hochreinen Präparat. Da der verwendete monoklonale Antikörper (mAk5.2) ein konformationelles Epitop im C-terminalen Bereich erkannte, wird durch diese 2-Stufenreinigung auf intaktes Protein voller Länge, mit korrekter Faltung zumindest am C-Terminus, setektioniert (Abb. 48).

Das Endprodukt besitzt, im Gegensatz zu dem natürlichen Material, am N-Terminus 11 zusätzliche Aminosäuren, davon 6 Histidine. Es enthält keine N-terminale Signal-und auch keine C-terminale Anker-Sequenz. Die *P. falciparum*-spezifische Sequenz beginnt mit Aminosäure 20 und endet mit Aminosäure 1621.

### 2. Kontrollierte Expression des gp190^{S1} in HeLa- und CHO-Zellkulturen.

Das gp190^{S1} wurde in den Vektor pBi-5 eingesetzt und damit unter Kontrolle eines durch Tetrazyklin (Tc) regulierbaren Promotors gestellt. Das Tc-kontrollierte System wurde aus 2 Gründen gewählt:
- Es gehört zu den Expressionssystemen, mit denen höchste Ausbeuten in Säugerzellen erreicht werden.
- Nicht-sekretierte Fremdproteine in hoher Konzentration können mit dem Metabolismus der Zellen in negativer Weise interferieren. Die Synthese des gewünschten Produktes wird daher erst nach aufwachsen der Kultur initiiert.

In dem Konstrukt pBi5-gp190^{S1} wurde ein bidirektionaler Promotor durch Tc-kontrollierten Transkriptionsaktivator (tTA) aktiviert und intitiierte die Transkription sowohl des gp190^{S1} als auch des Luziferase-Indikatorgens. In Anwesenheit von Tc ist der Promotor inaktiv. Die Transkriptionseinheit wurde sowohl in HeLa als auch in CHO-Zellen, welche beide konstitutiv tTA synthetisieren (HtTA-1-Linie (Gossen, M. and Bujard, H. (1992), Tight control of gene expression in mammalian cells by tetracycline-responsive promotors. Proc. Natl. Acad. Sci. USA 89, 5547-5551); CHO-tTA-Linie, unveröffentlicht), überführt. Durch Kotransfektion (Ca²⁺-Phosphat-Methode) mit einem Hygromycin-Resistenz-vermittelnden Markergen wurde auf erfolgreiche chromosomale Integration selektioniert. Hygromycin-resistente Klone wurden dann auf Regulierbarkeit der Expression ≥ Tc untersucht, indem die Luziferaseaktivität als Indikator genutzt wurde. In gut regulierbaren Klonen (Regulationsfaktor ≤ Tc 1000) wurde die gp190 Synthese geprüft. Immunfluoreszenz-Analyse (Abb. 5B) sowie Untersuchungen über "Western blot" (Abb. 5C) erlaubten, von beiden Zelltypen Klone zu identifizieren, welche gp190 unter streng regulierbaren Bedingungen synthetisieren. Von 20 Klonen wurde jeweils der bestregulierbare subkloniert. Die Subklone HtTA93-9 sowie CH027-29 wurden für Kulturen im 10 l Maßstab verwendet. Aus Zellextrakten dieser Kulturen ließ sich intaktes gp190^{S1} mittels Affinitätschromatographie (mAk5.2) isolieren. Das Material ist homogen bis auf eine einzige zelluläre Komponente, die nicht von gp190^{S1} abstammt und die etwa 25% des Präparats ausmacht (Abb. 6C). Sie müßte in einem weiteren Reinigungsschritt entfernt werden.

### 3. Expression des gp190^{S} in Toxoplasma gondii.

Toxoplasma gondii gehört wie *P*. *falciparum* zu den Apicomplexa und hat daher wahrscheinlich ein dem *P. felciparum* ähnliches Protein-Modifikationssystem. T. gondii läßt sich mit Fremd-DNA transfizieren, die effizient in das Genom integriert wird, außerdem läßt sich T. gondii problemlos in Zellkultur vermehren. Um ein möglichst natives gp190-Produkt zu erhalten, wurde gp190^{S2} so exprimiert, daß das Protein sekretiert und auf der Oberfläche des Parasiten, wie bei *P. falciparum*, über ein GPI-Motiv in der Membran verankert wird. Dazu wurde das gp190^{S2} (Abb. 3A) in das Plasmid ppTMCS (D. Soldati, unveröffentlicht) eingesetzt (Abb. 6A) und damit unter Kontrolle des T. gondii-Tubulin Promotors gestellt.

Dieses Expressionskonstrukt wurde in T. gondii transfiziert. Selektion mit Chloramphenicol führte zu resistenten Klonen, die gp190 synthetisieren, wie durch Immunfluoreszenz nachgewiesen wurde (Abb. 6B). Die Immunfluoreszenz mit anti-gp190-Antikörpern war nicht unterscheidbar von einer entsprechenden Anfärbung der Parasiten mittels Antikörper gegen SAG1, dem Hauptoberflächenprotein von T. gondii. Es ist daher davon auszugehen, daß gp190 an der Oberfläche von T. gondii verankert ist. Mehrere T. gondii-Klone (Nr. 3.1 bis 3.4) wurden charakterisiert und für die Produktion von gp190 aufbewahrt. Aus in präparativem Maßstab aufgewachsenen T. gondii-Kulturen (Klon 3.4) wurde gp190 mittels Affinitätschromatographie (mAK5.2.-Säule) isoliert. Analyse im elektrischen Feld zeigte ein homogenes Produkt mit einer Wanderungsgeschwindigkeit, die auf das intakte Protein schließen läßt (Abb. 6C).

### Beispiel 7: Charakterisierung von gp190 Protein aus verschiedenen Expressionssystemen mittels monoklonaler Antikörper.

Ein Satz gp190-spezifischer monoklonaler Antikörper, von denen mehrere konformationelle Epitope erkennen, wurde dazu benutzt, über Immunfluoreszenz die Reaktivität der Antikörper mit *P. falciparum-* bzw. T gondii-Parasiten zu vergleichen. Tabelle 1 zeigt, daß die Reaktivität der 16 Antikörper für beide Parasiten gleich ist. Dies ist ein starker Hinweis darauf, daß in T. gondii weitestgehend "natives" gp190 produziert wird. Der Vergleich der Reaktivität der Antikörper mit Protein aus *E. coli*, HeLa- bzw. CHO-Zellen sowie T. gondii zeigt ebenfalls, daß die meisten Antikörper mit den 4 Präparaten reagieren. Insbesondere wird das aus *E*. *coli* isolierte Protein von mehr Antikörpern erkannt als das Produkt aus Säugerzellen. Dies ist wahrscheinlich eine Konsequenz der Glykosylierung in Säugerzellen.

### Beispiel 8: Immunsierung von Aotus lemurinus griseimembra-Affen

### mit gp190/MSP1 aus P. falciparum (FCB-1).

Zwei unabhängige Immunsierungsexperimente (A, B) wurden durchgeführt. Dazu wurde aus jeweils ca. 2 x 10¹¹ Parasiten unter schonenden Bedingungen einmal 1,0 mg (A) und einmal 0,6 mg hochreines gp190/MSP1 isoliert.

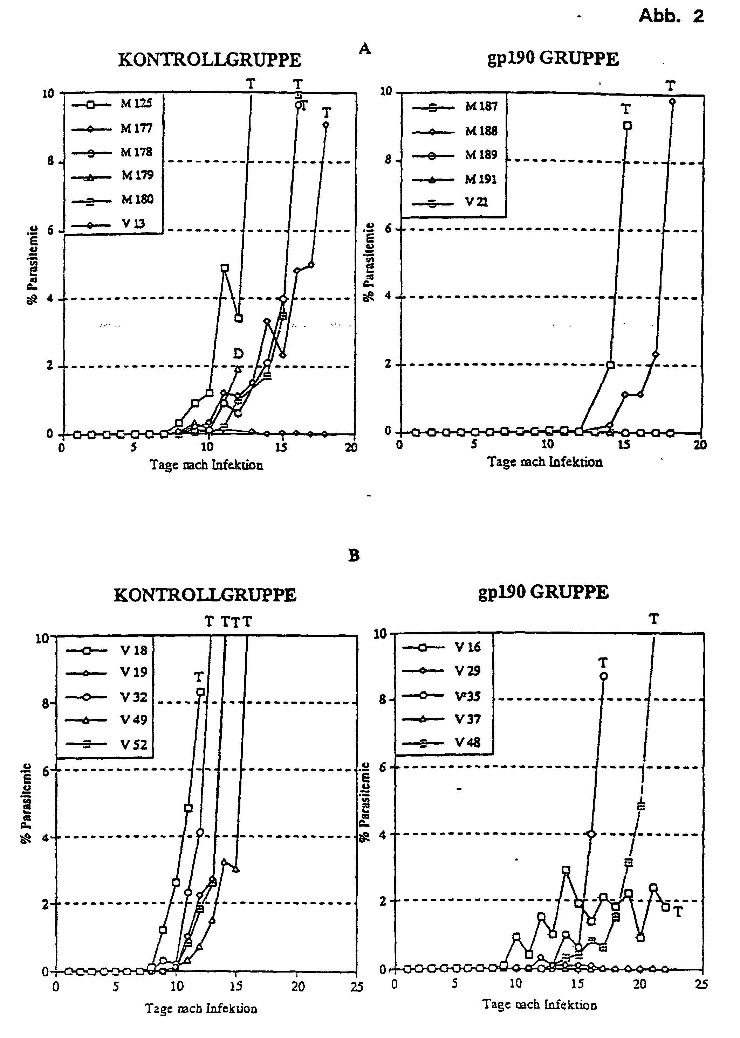

Das Protein wurde zusammen mit Freund'schem Adjuvans (FCA) verabreicht. Die Kontrollgruppe erhielt lediglich FCA. Es wurde dreimal im Abstand von 4 Wochen mit gleicher Menge an Protein bzw. mit Adjuvans immunisiert. Zwei Wochen nach der letzten Immunisierung wurden die Tiere mit jeweils 10⁵ Parasiten (FVO-Stamm) aus einem Donor-Tier infiziert. Die Parasitämie wurde täglich gemessen. Die Ergebnisse sind in Abb. 2 zusammengefaßt. Dabei bedeutet
T: daß die Tiere mit Resochin behandelt wurden
D: ein verstorbenes Tier
Abb. 2A.: die Individuen der geimpften Gruppe erhielten je 3 x 60 Mikrogramm gp190/MSP1
Abb. 2B: die Individuen der geimpften Gruppe erhielten je 3 x 40 Mikrogramm gp190/MSP1

Während in den Kontrollgruppen nur 1/11 Tiere keine Parasitämie entwickelten, waren es in der geimpften Gruppe 6/10. Die vier Tiere aus der geimpften Gruppe, die eine hohe Parasitämie entwickelten, taten dies - im Vergleich zur Kontrollgruppe - mit einer durchschnittlichen Verzögerung von vier Tagen (Überschreiten der 2%-Grenze der Parasitämie).

Diese Experimente zeigten erstmals einen hochsignifikanten Schutz gegen Infektion mit *P*. *falciparum* durch gp190/MSP1 im Affenmodell. Das erfindungsgemäße Verfahren erlaubt es somit erstmalig, einen wirksamen Impfstoff gegen die Malaria anzugeben.

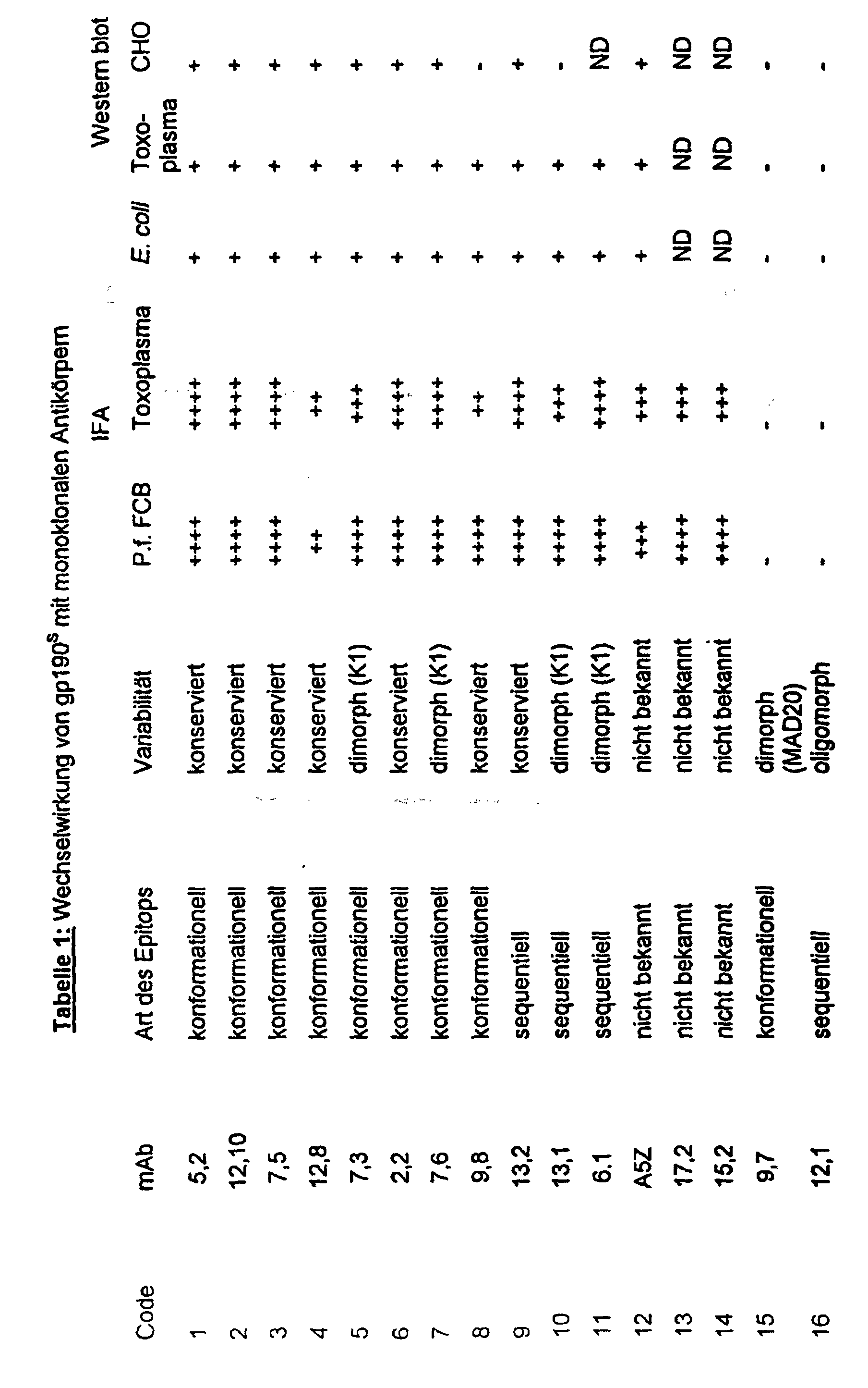

## Patentansprüche

1. Herstellungsverfahren für das vollständige gp190/MSP1-Protein von Plasmodium, insbesondere *Plasmodium falciparum*, **dadurch gekennzeichnet, daß** das vollständige Gen für gp190/MSP1 in einem geeigneten System, vorzugsweise einem Wirtsorganismus, exprimiert wird, und daß der AT-Gehalt der dem Protein zugrunde liegenden, exprimierten DNA-Sequenz gegenüber der natürlich vorkommenden Sequenz erniedrigt ist

2. Rekombinantes Herstellungsverfahren gemäß Anspruch 1, **dadurch gekennzeichnet, daß** der Synthese der dem Protein zugrunde liegenden DNA-Sequenz die DNA-Sequenz des *P. falciparum*-Stammes FCB-1 zugrunde gelegt wird.

3. Rekombinantes Herstellungsverfahren gemäß Anspruch 2, **dadurch gekennzeichnet, daß** der AT-Gehalt von 74% auf 55% erniedrigt ist.

4. Rekombinantes Herstellungsverfahren gemäß einem oder mehreren der Ansprüche 1 bis 3, **dadurch gekennzeichnet, daß** das dem erzeugten Protein zugrunde liegende Gen für die volle Aminosäuresequenz einschließlich Signalpeptid und Ankersignal kodiert.

5. Rekombinantes Herstellungsverfahren gemäß einem oder mehreren der Ansprüche 1 bis 3, **dadurch gekennzeichnet, daß** das dem erzeugten Protein zugrunde liegende Gen für die volle Aminosäuresequenz mit Ausnahme des Ankersignals kodiert.

6. Rekombinantes Herstellungsverfahren gemäß einem oder mehreren der Ansprüche 1 bis 3, **dadurch gekennzeichnet, daß** das dem erzeugten Protein zugrunde liegende Gen für die volle Aminosäuresequenz mit Ausnahme des Ankersignals und des Signalpeptides kodiert.

7. Rekombinantes Herstellungsverfahren gemäß einem oder mehreren der Ansprüche 1 bis 6, **dadurch gekennzeichnet, daß** es folgende Schritte umfaßt
(a) Entwurf der zu synthetisierenden DNA-Sequenz aus *P. falciparum* FCB-1, wobei eine DNA-Sequenz mit den im menschlichen Genom üblichen Codon-Häufigkeiten unter Erhalt der Aminosäuresequenz des FCB-1 Proteins hergestellt werden sollte,
(b) Einteilung der entworfenen Sequenz in überlappende Regionen, vorzugsweise in Regionen p83, p31, p36, p30 und p19,
(c) Synthese von Desoxyoligonukleotiden, die jeweils mindestens die gesamte Länge einer Region abdecken,
(d) Synthese der kodierenden Regionen für p19, p30, p36 und p31 durch PCR und Synthese der kodierenden Region für p83 durch Fusion aus zwei etwa 1200bp umfassenden Sequenzen,
(e) einzelne Klonierung der kodierenden Sequenzen
(f) Fusion des gesamten Gens und
(g) Expression in einem geeigneten Expressionssystem.

8. Rekombinantes Herstellungsverfahren nach Anspruch 7, **dadurch gekennzeichnet, daß** die in Schritt (c) synthetisierten Desoxyoligonukleotide durchschnittlich 120 Nukleotide lang sind und die benachbarten Sequenzen jeweils um ca 20 Basen überlappen.

9. Rekombinantes Herstellungsverfahren nach einem oder mehreren der Ansprüche 1 bis 8, **dadurch gekennzeichnet, daß** als Expressionsvektor pDS56, RBSII verwendet wird.

10. Rekombinantes Herstellungsverfahren nach einem oder mehreren der Ansprüche 1 bis 8, **dadurch gekennzeichnet, daß** als Expressionsvektor pBi-5 verwendet wird.

11. Rekombinantes Herstellungsverfahren nach einem oder mehreren der Ansprüche 1 bis 8, **dadurch gekennzeichnet, daß** als Expressionsvektor ppTMCS vewendet wird.

12. Rekombinantes Herstellungsverfahren nach einem oder mehreren der Ansprüche 1 bis 11, **dadurch gekennzeichnet, daß** die dem Protein zugrunde liegende DNA-Sequenz in *E. coli* exprimiert wird.

13. Rekombinantes Herstellungsverfahren nach Anspruch 12, **dadurch gekennzeichnet, daß** der verwendete *E. coli*-Stamm der Repressor-produzierende Stamm *E. coli* DH5alphaZ1 ist.

14. Rekombinantes Herstellungsverfahren nach einem oder mehreren der Ansprüche 1 bis 11, **dadurch gekennzeichnet, daß** die dem Protein zugrunde liegende DNA-Sequenz in HeLa-Zellen exprimiert wird.

15. Rekombinantes Herstellungsverfahren nach einem oder mehreren der Ansprüche 1 bis 11, **dadurch gekennzeichnet, daß** die dem Protein zugrunde liegende DNA-Sequenz in CHO-Zellen exprimiert wird.

16. Rekombinantes Herstellungsverfahren nach einem oder mehreren der Ansprüche 1 bis 11, **dadurch gekennzeichnet, daß** die dem Protein zugrunde liegende DNA-Sequenz in *Toxoplasma gondii* oder *Leishmania* exprimiert wird.

17. Vollständige, zur Expression geeignete DNA-Sequenz des gp190/MSP1-Oberflächenproteins von Plasmodium, insbesondere *P*. *falciparum*, erhältlich durch das rekombinante Herstellungsverfahren gemäß einem oder mehreren der Ansprüche 1 bis 16.

18. Zur Expression geeignete DNA-Sequenz gemäß Anspruch 17, **dadurch gekennzeichnet, daß** sie nicht für das Ankersignal kodiert.

19. Zur Expression geeignete DNA-Sequenz gemäß Anspruch 18, **dadurch gekennzeichnet, daß** sie weder für das Ankersignal noch für das Signalpeptid kodiert.

20. Zur Expression geeignete DNA-Sequenz gemäß Anspruch 19, **dadurch gekennzeichnet, daß** sie eine Sequenz für am N-Terminus vorliegende 11 zusätzliche Aminosäuren, davon 6 Histidine, umfaßt.

21. Zur Expression geeignete DNA-Sequenz gemäß einem oder mehreren der Ansprüche 17 bis 20, **dadurch gekennzeichnet, daß** die Sequenz keine erkennbaren "splice donor"- und "splice acceptor"-Signale enthält.

22. Zur Expression geeignete DNA-Sequenz gemäß einem oder mehreren der Ansprüche 17 bis 21, **dadurch gekennzeichnet, daß** die Sequenz keine größeren GC-reichen Sequenzen enthält.

23. Zur Expression geeignete DNA-Sequenz gemäß einem oder mehreren der Ansprüche 17 bis 22, **dadurch gekennzeichnet, daß** die Sequenz keine Erkennungssignale für Restriktionsenzyme, weiche Sequenzen von sechs oder mehr Basenpaaren erkennen, enthält.

24. Zur Expression geeignete DNA-Sequenz gemäß einem oder mehreren der Ansprüche 17 bis 23, **dadurch gekennzeichnet, daß** die Sequenz für Erkennungssignale bestimmter Restriktionsnukleasen in Regionen, die die nach der Prozessierung des Proteins entstehenden Domänen trennen, einmal vorkommende Schnittstellen für Restriktionsendonukleasen enthält.

25. Zur Expression geeignete DNA-Sequenz gemäß einem oder mehreren der Ansprüche 17 bis 24, **dadurch gekennzeichnet, daß** die Sequenz an ihren beiden Enden Schnittstellen für Restriktonsendonukleasen aufweist, die in der übrigen Sequenz und in einem zu verwendenden Vektor nicht vorkommen.

26. Wirtsorganismus, der die vollständige Nukleinsäuresequenz gemäß einem der Ansprüche 17 bis 25 für das gp190/MSP1-Oberflächenprotein enthält.

27. Wirtsorganismus gemäß Anspruch 26, **dadurch gekennzeichnet, daß** der Wirtsorganismus *E. coli* ist.

28. Wirtsorganismus gemäß Anspruch 27, **dadurch gekennzeichnet, daß** der *E. coli*-Stamm der Repressor-produzierende *E. coli*-Stamm DH5alphaZ1 ist.

29. Wirtsorganismus gemäß Anspruch 26, **dadurch gekennzeichnet, daß** der Wirtsorganismus HeLa-Zellen sind.

30. Wirtsorganismus gemäß Anspruch 26, **dadurch gekennzeichnet, daß** der Wirtsorganismus CHO-Zellen sind.

31. Wirtsorganismus gemäß Anspruch 29 oder 30, **dadurch gekennzeichnet, daß** die Wirtszellen konstitutiv tTA synthetisieren.

32. Wirtsorganismus gemäß Anspruch 26, **dadurch gekennzeichnet, daß** als Wirtsorganismus *Toxoplasma gondli, Leishmania*, Baculoviren, Adenoviren oder Hefen vorgesehen sind.

33. Verwendung eines gemäß einem der Ansprüche 1 bis 16 hergestellten gp190/MSP1-Proteins zur aktiven Immunisierung gegen Malaria.

34. Verwendung eines gemäß einem der Ansprüche 1 bis 16 hergestelten gp190/MSP1-Proteins zur Herstellung von monoklonalen, zur passiven Immunisierung geeigneten Antikörpern.

35. Verwendung einer gemäß der Ansprüche 1 bis 16 hergestellten DNA-Sequenz zur Herstellung einer Vakzine auf Nukleinsäurebasis.

36. Vektor enthaltend eine DNA-Sequenz nach einem der Ansprüche 17 bis 25.

37. Wirtszelle enthaltend einen Vektor nach Anspruch 36.

38. Impfstoff enthaltend eine DNA-Sequenz nach einem der Ansprüche 17-25 und/oder einen Wirt nach einem der Ansprüche 26-31 und/oder einen Vektor nach Anspruch 36.

39. Impfstoff nach Anspruch 38, **dadurch gekennzeichnet, daß** er weitere Immunität hervorrufende Produkte aus Plasmodium, insbesondere *P. falciparum,* enthält

## Claims

1. Process for preparing of the complete gp190/MSP1 protein of Plasmodium, in particular Plasmodium falciparum, **characterized in that** the complete gene for gp190/MSP1 is expressed in a suitable system, preferably a host organism, and that the AT content of the expressed DNA sequence on which the protein is based is reduced compared to the naturally occurring sequence.

2. Recombinant process according to Claim 1, **characterized in that** the synthesis of the DNA sequence on which the protein is based derives from the DNA sequence of the P. falciparum strain FCB-1.

3. Recombinant process according to Claim 2, **characterized in that** the AT content is reduced from 74% to 55%.

4. Recombinant process according to one or more of claims 1 to 3, **characterized in that** the gene on which the protein produced is based codes for the complete amino-acid sequence including signal peptide and attachment signal.

5. Recombinant process according to one or more of claims 1 to 3, **characterized in that** the gene on which the protein produced is based codes for the complete amino-acid sequence except for the attachment signal.

6. Recombinant process according to one or more of claims 1 to 3, **characterized in that** the gene on which the protein produced is based codes for the complete amino-acid sequence except for the attachment signal and the signal peptide.

7. Recombinant process according to one or more of claims 1 to 6, **characterized in that** it includes the according to steps:
(a) Design of the DNA sequence from P. falciparum FCB-1 to be synthesized, by which a DNA sequence with the codon frequencies common in the human genome and maintaining the amino-acid sequence of the FCB-1 protein would be produced,
(b) division of the planned sequence into overlapping regions, preferably regions p83, p31, p36, p30and p19,
(c) synthesis of desoxyoligonucleotides, each of them extending the whole length of a region,
(d) synthesis of the regions coding for p19, ip30, p36 and p31 by PCR and synthesis of the region coding for p83 by fusion of two sequences comprising approximately 1200 bp,
(e) individual cloning of coding sequences,
(f) fusion of the complete gene and
(g) expression in a suitable expression system.

8. Recombinant process according to Claim 7, **characterized in that** the desoxyoligonucleotides synthesized in step (c) should be on average 120 nucleotides long and in each instance overlap the neighbouring sequences by about 20 bases.

9. Recombinant process according to one or more of Claims 1 to 8, **characterized in that** pD556 used as expression vector.

10. Recombinant manufacturing process according to one or more of Claims 1 to 8, **characterized in that** pBi-5 is used as expression vector.

11. Recombinant process according to one or more of Claims 1 to 8, **characterized in that** ppTMCS is used as expression vector.

12. Recombinant process according to one or more of Claims 1 to 11, **characterized in that** the DNA sequence on which the protein is based is expressed in E. coli.

13. Recombinant process according to Claim 12, **characterized in that** the E.coli strain used is the repressor-producing strain E. coli DH5alphaZ1.

14. Recombinant process according to one or more of Claims 1 to 11, **characterized in that** the DNA sequence on which the protein is based is expressed in HeLa cells.

15. Recombinant process according to one or more of Claims 1 to 11, **characterized in that** the DNA sequence on which the protein is based is expressed in CHO cells.

16. Recombinant process according to one or more of Claims 1 to 11, **characterized in that** the DNA sequence on which the protein is based is expressed in Toxoplasma gondii or Leishmania.

17. Complete DNA sequence, suitable for expression, of the gp190/MSP1 surface protein of Plasmodium, in particular P. falciparum, obtainable by the recombinant process according to one or more of Claims 1 to 16.

18. DNA sequence, suitable for expression, according to Claim 17, **characterized in that** it does not code for the attachment signal.

19. DNA sequence, suitable for expression, according to Claim 18, **characterized in that** it codes neither for the attachment signal nor the signal peptide.

20. DNA sequence, suitable for expression, according to Claim 19, **characterized in that** it includes a sequence for 11 additional amino-acids, of which 6 are histidines, present at the N-terminus.

21. DNA sequence, suitable for expression, according to one or more of Claims 17 to 20, **characterized in that** the sequence includes no recognizable "splice donor" and "splice acceptor" signals.

22. DNA sequence, suitable for expression, according to one or more of Claims 17 to 21, **characterized in that** the sequence includes no large GC-rich sequences.

23. DNA sequence, suitable for expression, according to one or more of Claims 17 to 22, **characterized in that** the sequence includes no recognition signals for restriction enzymes recognizing sequences of six or more base pairs.

24. DNA sequence, suitable for expression, according to one or more of Claims 17 to 23, **characterized in that** the sequence for recognition signals of particular restriction nucleases in regions which, after processing of the protein, separate existing domains, contains uniquely occurring cleavage sites for restriction endonucleases.

25. DNA sequence, suitable for expression, according to one or more of Claims 17 to 24, **characterized in that** the sequence has at both its ends cleavage sites for endonucleases which do not appear in the remaining sequence and in a vector for utilization.

26. Host organism which contains the complete nucleic acid sequence according to any of Claims 17 to 25 for the gp190/MSP1 surface protein*

27. Host organism according to Claim 26, **characterized in that** the organism is E. coli.

28. Host organism according to Claim 27, **characterized in that** the E. coli strain is the repressor-producing E. coli strain DH5alphaZ1.

29. Host organism according to Claim 26, **characterized in that** the host organism is HeLa cells.

30. Host organism according to Claim 26, **characterized in that** the host organism is CHO cells.

31. Host organism according to Claim 29 or 30, **characterized in that** the host cells synthesize constitutively tTA.

32. Host organism according to Claim 26, **characterized in that** the host organisms provided are Toxoplasma gondii, Leishmania, baculoviruses, adenoviruses or yeasts.

33. Use of a gp190/MSP1 protein manufactured according to Claims 1 to 16 for active immunization against malaria.

34. Use of a gp190/MSP1 protein manufactured according to Claims 1 to 16 for the manufacture of monoclonal antibodies suitable for passive immunization.

35. Use of a DNA sequence manufactured according to Claims 1 to 16 for the manufacture of a vaccine based on nucleic acids.

36. Vector containing DNA sequence according to one of claims 17 to 25.

37. Host cell containing a vector according to claim 36.

38. Vaccine containing a DNA sequence according to one of claims 17 to 25 and/or a host according to one of claims 26 to 31 and/or a vector according to claim 36.

39. Vaccine according to claim 38, **characterized in that** it contains further immunity-promoting products from Plasmodium, especially P. falciparum.

## Revendications

1. Procédé de préparation de la protéine gp190/MSP1 entière d'un plasmodium, en particulier de *Plasmodium falciparum,* **caractérisé en ce que** le gène entier de gp190/MSP1 est exprimé dans un système approprié, de préférence un organisme hôte, et **en ce que** la teneur en AT de la séquence d'ADN qui se trouve à la base de la protéine et qui est exprimée dans la protéine est abaissée par rapport à la séquence d'origine naturelle.

2. Procédé de préparation recombinante selon la revendication 1, **caractérisé en ce que** la synthèse de la séquence d'ADN qui se trouve à la base la protéine est basée sur la séquence d'ADN de la souche FCB-1 de *P. falciparum*.

3. Procédé de préparation recombinante selon la revendication 2, **caractérisé en ce que** la teneur en AT est abaissée de 74% à 55%.

4. Procédé de préparation recombinante selon une ou plusieurs des revendications 1 à 3, **caractérisé en ce que** le gène à la base de la protéine produite code la totalité de la séquence d'acides aminés, y compris le peptide signal et le signal d'ancrage.

5. Procédé de préparation recombinante selon une ou plusieurs des revendications 1 à 3, **caractérisé en ce que** le gène à la base de la protéine produite code la totalité de la séquence d'acides aminés, à l'exception du signal d'ancrage.

6. Procédé de préparation recombinante selon une ou plusieurs des revendications 1 à 3, **caractérisé en ce que** le gène à la base de la protéine produite code la totalité de la séquence d'acides aminés, à l'exception du signal d'ancrage et du peptide signal.

7. Procédé de préparation recombinante selon une ou plusieurs des revendications 1 à 6, **caractérisé en ce qu'**il comprend les étapes suivantes :
(a) mise au point de la séquence d'ADN à synthétiser à partir de FCB-1 de *P. falciparum*, moyennant quoi une séquence d'ADN ayant les fréquences de codons habituelles dans le génome humain doit être produite tout en conservant la séquence d'acides aminés de la protéine de FCB-1,
(b) répartition de la séquence mise au point dans des régions chevauchantes, de préférence dans les régions p83, p31, p36, p30 et p19,
(c) synthèse de désoxyoligonucléotides qui recouvrent chacun au moins la longueur entière d'une région,
(d) synthèse des régions codant p19, p30, p36 et p31 par PCR et synthèse de la région codant p83 par fusion de deux séquences contenant environ 1200 paires de base,
(e) clonage individuel des séquences codantes
(f) fusion du gène entier et
(g) expression dans un système d'expression approprié.

8. Procédé de préparation recombinante selon la revendication 7, **caractérisé en ce que** les désoxyoligonucléotides synthétisés dans l'étape (c) sont longs, en moyenne, de 120 nucléotides et chevauchent les séquences voisines à chaque fois sur environ 20 bases.

9. Procédé de préparation recombinante selon une ou plusieurs des revendications 1 à 8, **caractérisé en ce que** l'on utilise pDS56, RBSII en tant que vecteur d'expression.

10. Procédé de préparation recombinante selon une ou plusieurs des revendications 1 à 8, **caractérisé en ce que** l'on utilise pBi-5 en tant que vecteur d'expression.

11. Procédé de préparation recombinante selon une ou plusieurs des revendications 1 à 8, **caractérisé en ce que** l'on utilise ppTMCS en tant que vecteur d'expression.

12. Procédé de préparation recombinante selon une ou plusieurs des revendications 1 à 11, **caractérisé en ce que** la séquence d'ADN à la base de la protéine est exprimée dans *E. coli.*

13. Procédé de préparation recombinante selon la revendication 12, **caractérisé en ce que** la souche d'*E. coli* utilisée est la souche produisant un répresseur, DH5alphaZ1 d'*E. coli.*

14. Procédé de préparation recombinante selon une ou plusieurs des revendications 1 à 11, **caractérisé en ce que** la séquence d'ADN à la base de la protéine est exprimée dans des cellules HeLa.

15. Procédé de préparation recombinante selon une ou plusieurs des revendications 1 à 11, **caractérisé en ce que** la séquence d'ADN à la base de la protéine est exprimée dans des cellules CHO.

16. Procédé de préparation recombinante selon une ou plusieurs des revendications 1 à 11, **caractérisé en ce que** la séquence d'ADN à la base de la protéine est exprimée dans *Toxoplasma gondii* ou *Leishmania.*

17. Séquence d'ADN entière, appropriée pour l'expression, de la protéine de surface gp190/MSP1 d'un plasmodium, en particulier de *P. falciparum*, susceptible d'être obtenue par le procédé de préparation recombinante selon une ou plusieurs des revendications 1 à 16.

18. Séquence d'ADN appropriée pour l'expression selon la revendication 17, **caractérisée en ce qu'**elle ne code pas le signal d'ancrage.

19. Séquence d'ADN appropriée pour l'expression selon la revendication 18, **caractérisée en ce qu'**elle ne code ni le signal d'ancrage, ni le peptide signal.

20. Séquence d'ADN appropriée pour l'expression selon la revendication 19, **caractérisée en ce qu'**elle comprend une séquence pour 11 acides aminés supplémentaires se trouvant à l'extrémité N, dont 6 histidines.

21. Séquence d'ADN appropriée pour l'expression selon une ou plusieurs des revendications 17 à 20, **caractérisée en ce que** la séquence ne contient pas de signaux de "splice donor" et de "splice acceptor" reconnaissables.

22. Séquence d'ADN appropriée pour l'expression selon une ou plusieurs des revendications 17 à 21, **caractérisée en ce que** la séquence ne contient pas de séquences riches en GC plus grandes.

23. Séquence d'ADN appropriée pour l'expression selon une ou plusieurs des revendications 17 à 22, **caractérisée en ce que** la séquence ne contient pas de signaux de reconnaissance pour des enzymes de restriction, lesquelles reconnaissent des séquences de six paires de bases ou plus.

24. Séquence d'ADN appropriée pour l'expression selon une ou plusieurs des revendications 17 à 23, **caractérisée en ce que** la séquence contient des sites de coupure, pour des endonucléases de restriction, n'apparaissant qu'une fois, pour des signaux de reconnaissance de certaines nucléases de restriction dans des régions, qui séparent les domaines formés après la synthèse de la protéine.

25. Séquence d'ADN appropriée pour l'expression selon une ou plusieurs des revendications 17 à 24, **caractérisée en ce que** la séquence présente, à ses deux extrémités, des sites de coupure pour des endonucléases de restriction, qui n'existent pas dans le reste de la séquence ni dans un vecteur à utiliser.

26. Organisme hôte qui contient la séquence d'acide nucléique entière selon l'une des revendications 17 à 25 pour la protéine de surface gp190/MSP1.

27. Organisme hôte selon la revendication 26, **caractérisé en ce que** l'organisme hôte *est E. coli*.

28. Organisme hôte selon la revendication 27, **caractérisé en ce que** la souche d'*E. coli* utilisée est la souche produisant un répresseur DH5alphaZ1 d'*E. coli*.

29. Organisme hôte selon la revendication 26, **caractérisé en ce que** les organismes hôtes sont des cellules HeLa.

30. Organisme hôte selon la revendication 26, **caractérisé en ce que** les organismes hôtes sont des cellules CHO.

31. Organisme hôte selon la revendication 29 ou 30, **caractérisé en ce que** les cellules hôtes synthétisent constitutivement tTA.

32. Organisme hôte selon la revendication 26, **caractérisé en ce que** l'on prévoit, en tant qu'organisme hôte, *Toxoplasma gondii, Leishmania,* des bacillovirus, des adénovirus ou des levures.

33. Utilisation d'une protéine gp190/MSP1 préparée selon l'une des revendications 1 à 16 pour l'immunisation active contre le paludisme.

34. Utilisation d'une protéine gp190/MSP1 préparée selon l'une des revendications 1 à 16 pour la production d'anticorps monoclonaux appropriés pour une immunisation passive.

35. Utilisation d'une séquence d'ADN préparée selon les revendications 1 à 16 pour la production d'un vaccin à base d'acide nucléique.

36. Vecteur contenant une séquence d'ADN selon l'une des revendications 17 à 25.

37. Cellule hôte contenant un vecteur selon la revendication 36.

38. Vaccin contenant une séquence d'ADN selon l'une des revendications 17 à 25 et/ou un hôte selon l'une des revendications 26 à 31 et/ou un vecteur selon la revendication 36.

39. Vaccin selon la revendication 38, **caractérisé en ce que** qu'il contient d'autres produits immunisants provenant d'un plasmodium, en particulier de *P. falciparum*.
